# FASCICULE DE BREVET EUROPEEN

(11) **EP 1 627 395 B1**
(45) Date de publication et mention de la délivrance du brevet: **15.02.2012**
(21) Numéro de dépôt: 04742722.4
(22) Date de dépôt: 13.05.2004
(51) Int. Cl.: H01F 1/44, H01F 1/00, A61K 49/18

(54) **DISPERSIONS AQUEUSES STABLES EN MILIEU NEUTRE, COMPRENANT DES PARTICULES À SURFACE MODIFIÉE**
IN NEUTRALEN MEDIEN STABILE WÄSSRIGE DISPERSIONEN MIT OBERFLÄCHENMODIFIZIERTEN TEILCHEN
AQUEOUS DISPERSIONS STABLE IN NEUTRAL MEDIA, COMPRISING SURFACE-MODIFIED PARTICLES

(30) Priorité: 23.05.2003 FR 0306279
(43) Date de publication de la demande: 22.02.2006
(73) Titulaire: CENTRE NATIONAL DE LA RECHERCHE SCIENTIFIQUE (CNRS), 75016 Paris (FR)
(72) Inventeur: DUGUET, Etienne, F-33130 Begles (FR); MORNET, Stéphane, F-33370 Artigues pres Bordeaux (FR); PORTIER, Joseph, F-33170 Gradignan (FR)
(74) Mandataire: Colombet, Alain André
(86) Numéro de dépôt international: PCT/FR2004/001169
(87) Numéro de publication internationale: WO 2004/107368

(56) Documents cités:
- WO-A-99/62079
- US-A- 4 965 007
- US-A- 5 262 176
- US-A- 5 776 360

## Description

La présente invention a trait à des dispersions aqueuses de particules de dimensions nanométriques à base d'oxyde de fer magnétique, de surface modifiée, qui présentent un très faible taux d'agglomération interparticulaire, notamment en milieu neutre. Ces dispersions de particules magnétiques de surface modifiée et essentiellement individualisées se révèlent utiles notamment pour la préparation de compositions d'intérêt diagnostique ou thérapeutique, et tout particulièrement pour la préparation de compositions adaptées à une administration par injection.

On connaît actuellement différents types de compositions comprenant des particules magnétiques nanométriques (de dimensions inférieures à 100nm), dispersées au sein de milieux dispersants liquides de nature aqueuse (eau ou solutions aqueuses), ou bien de nature organique (hydrocarbures, kérosène, xylène, toluène, polyglycols ou phényléthers, notamment).

Dans ces compositions, les particules les plus utilisées sont des particules à base de fer (Fe-α), de cobalt (Co-α), et des particules à base d'oxyde(s) de fer magnétique(s) tels que des ferrites (à savoir, le plus souvent, des oxydes de formule globale M^{II}Fe^{III}Fe^{II}O₄, dans lesquels M^{II} désigne un métal choisi parmi Fe, Co, Ni, Cd, Mn, Zn ou Mg, à son état d'oxydation +II).

Bien qu'elles présentent en général des propriétés magnétiques moins prononcées que les particules de Fe-α ou de Co-α, les particules à base d'oxydes de fer magnétiques (et notamment les particules à base de ferrites) se révèlent plus stables vis-à-vis de l'oxydation, et elles sont par conséquent plus largement utilisées, en particulier dans les dispersions aqueuses. Dans ce cadre, on utilise en particulier des particules ferrimagnétiques à base de magnétite (Fe₃O₄) ou de maghémite (Fe₂O₃-γ).

Les dispersions, en particulier aqueuses, de particules à base d'oxydes de fer magnétique sont souvent désignées par le terme générique de "ferrofluide". Cette dénomination vient du fait que, lorsque ce type de dispersion présente une concentration suffisamment importante en particules d'oxyde de fer, l'application d'un gradient de champ magnétique provoque la mise en déplacement des particules qui entraînent le liquide. A l'échelle macroscopique, le comportement observé est schématiquement celui d'un "liquide magnétique".

Un des problèmes à résoudre lorsqu'on synthétise un ferrofluide est celui de la stabilisation des particules magnétiques vis-à-vis de l'agglomération.

En effet, de façon générale, dans un ferrofluide (comme dans toute dispersion de particules d'oxyde de dimensions nanométriques), les particules à base d'oxyde tendent à s'attirer mutuellement, notamment du fait d'interactions attractives de type Van der Waals. Il s'avère donc nécessaire d'assurer une stabilisation de la dispersion, de façon à inhiber le phénomène naturel de floculation. De façon à inhiber le phénomène d'agglomération interparticulaire, on met en général en oeuvre, au sein des dispersions de type "ferrofluides", des particules présentant des charges de surface induisant des interactions répulsives entre les particules ("stabilisation électrostatique") ou des particules présentant en surface des macromolécules, adsorbées ou greffées ("stabilisation stérique"). Lorsque les deux modes de stabilisation sont utilisés conjointement, on parle de "stabilisation électrostérique".

La stabilisation d'un ferrofluide s'avère d'autant plus difficile à réaliser que la taille des particules présentes est faible, et que la concentration en particules est élevée.

Or, notamment pour obtenir des propriétés magnétiques suffisamment prononcées, il est en général souhaitable que la concentration en particules soit élevée au sein d'un ferrofluide.

De plus, dans une dispersion de particules magnétiques de type "ferrofluide", il est particulièrement avantageux que les particules d'oxyde soient des particules de faibles dimensions, de préférence inférieure à 20 nm, et typiquement entre 3 et 15 nm. Dans ce cas, en effet, les particules sont en général monocristallines, et chacune des particules est alors une particule magnétique "monodomaine", possédant un moment magnétique propre et se comportant comme un macrospin magnétique. Le ferrofluide présente alors un comportement global de nature paramagnétique (phénomène dit de "superparamagnétisme"), c'est-à-dire que les particules ne conservent pas d'aimantation rémanente en l'absence de champ magnétique. Cette absence d'aimantation rémanente présente l'avantage de ne pas induire d'interactions magnétiques entre les particules de nature à provoquer leur agglomération, contrairement à ce qui serait observé avec des particules magnétiques non monocristallines, qui tendent à s'attirer les unes les autres pour compenser leurs moments magnétiques rémanents.

Il est également à noter que, lorsqu'on souhaite utiliser des dispersions de particules magnétiques pour une administration chez l'animal ou l'être humain, par exemple à titre d'agent de contraste pour l'IRM, il est souvent nécessaire de disposer de particules présentant un diamètre hydrodynamique le plus faible possible, notamment de façon à traverser le maximum de membranes biologiques. Lorsque les dispersions sont injectées par voie intraveineuse, la réduction de la taille des particules s'avère également nécessaire pour éviter les phénomènes d'obstruction du réseau sanguin, notamment au niveau des capillaires, en particulier pour limiter les risques d'embolies.

Un des moyens les plus usuels pour obtenir des dispersions aqueuses de particules d'un oxyde de fer magnétique présentant un taux d'agglomération interparticulaire le plus réduit possible consiste à contrôler le pH du milieu aqueux dispersant soit à une valeur inférieure à 5 (ferrofluide acide), soit à une valeur supérieure à 10 (ferrofluide basique).

A ce sujet, il a été observé qu'en milieu aqueux, on observe, à la surface de particules d'oxydes de fer telles que des ferrites une chimisorption de molécules d'eau qui conduit à la formation d'espèces hydroxyles qu'on peut schématiser par ≡Fe^{III}-OH. Ces espèces hydroxyles sont amphotères et, en fonction du pH du milieu aqueux, elles mènent à la formation de charges positives ou négatives selon les schémas réactionnels suivants :

≡Fe-OH + H₃O⁺ ⇔ ≡Fe-OH₂⁺ + H₂O (milieu acide)

≡Fe-OH + OH⁻ ⇔ ≡Fe-O⁻ + H₂O (milieu basique)

Toutefois, à des pH compris entre 5 et 10, la densité de charges présente à la surface de particules d'oxyde de fer est trop faible pour assurer une stabilisation de type électrostatique. A ce sujet, il est à noter que le point isoélectrique d'un oxyde de fer, à savoir le pH auquel les charges présentes en surface de l'oxyde de fer se compensent, est en général de l'ordre de 7 (il est généralement compris entre 6 et 9 et vaut typiquement 7 notamment dans le cas de la maghémite). Ainsi, il s'avère que la stabilité d'un ferrofluide aqueux (notamment de type USPIO) à un pH de l'ordre de 7 ne peut pas être obtenu par simple stabilisation électrostatique, ce qui constitue une limitation à l'utilisation des particules d'oxyde de fer de type ferrite notamment pour des applications médicales (agent de contraste pour IRM notamment) où il est justement nécessaire de disposer de dispersions stables à un pH physiologique, à savoir à un pH de l'ordre de 7,4.

Pour assurer la stabilité de ferrofluides aqueux de pH neutre, on a par conséquent développé des modes de stabilisation mettant en oeuvre un greffage de molécules à la surface des particules, dans le but de déplacer le point isoélectrique de la dispersion, et par conséquent le domaine de stabilité de cette dispersion vis-à-vis de l'agglomération interparticulaire.

Le "point isoélectrique" des particules d'un ferrofluide auquel il est fait référence dans la présente description désigne la valeur du pH de la dispersion pour laquelle les charge(s) surfacique(s) portée par lesdites particules se compensent. Ce point isoélectrique (ou PIE) peut notamment être déterminé par zêtamétrie, à savoir en mesurant le potentiel zêta d'une dispersion des particules à différentes valeurs de pH, par exemple selon la méthode décrite par R.J. Hunter dans Zeta Potential in Colloid Science, Principles and Applications, Academie Press, London (1986*).*

De façon générale, un ferrofluide est stable vis-à-vis de l'agglomération interparticulaire dans les domaines de pH éloignés du PIE, alors qu'à des pH de l'ordre du PIE, le ferrofluide devient instable. La zone d'instabilité peut être plus ou moins resserrée autour de la valeur du PIE, l'étendue de cette zone variant notamment en fonction de la taille des particules du ferrofluide et du nombre de sites chargés de surface. Ainsi, en général, l'étendue de la zone d'instabilité décroît avec la taille des particules. Dans la plupart des cas, un ferrofluide est stable à des valeurs de pH inférieures à (PIE-2) (et supérieures à (PIE+2)) mais la zone de stabilité du ferrofluide peut cependant varier de façon plus ou moins importante, En tout état de cause, un ferrofluide perd sa stabilité à un pH au voisinage du PIE. Pour obtenir des ferrofluides stables en milieu neutre, on a donc cherché à obtenir des ferrofluides ayant un PIE le plus éloigné possible de la valeur de 7. Dans ce cadre, on a notamment décrit des compositions de ferrofluide stabilisées en milieu neutre, présentant une surface modifiée par des espèces aminées, obtenues par greffage de molécules telles que des aminosilanes.

Le brevet US 5,776,360 décrit des dispersions aqueuses comprenant des particules magnétiques à base d'oxyde de fer avec un diamètre de 1 à 10 nm dont la surface est fonctionnalisée par des groupes silanes, qui sont liés de façon covalente. Ce brevet ne mentionne pas le point isoélectrique des particules divulguées.

Toutefois, les procédés développés dans ce but conduisent, le plus souvent, à des processus d'agglomération des particules au cours du greffage, et ils permettent de ce fait uniquement l'obtention d'agrégats particulaires greffés en surface. A titre de procédé de ce type, menant à l'obtention de particules de taille importante, on peut par exemple citer le procédé notamment décrit par Whitehead et al. dans le brevet US 4,695, 393.

Pour éviter le phénomène d'agrégation interparticulaire au cours du greffage par des espèces de type aminosilane, il a été proposé de conduire la réaction de greffage en soumettant le milieu réactionnel à un traitement sous ultrasons (sonication), dans le but d'obtenir une dispersion des particules d'oxyde au sein du milieu où est réalisé le greffage. A ce sujet, on pourra notamment se reporter à l'article de Lesniak et al. dans Mat. Res. Soc. Symp. Proc., 432, 169-174, (1997). Dans ces procédés, il est préconisé de conduire la sonication dans des

conditions les plus poussées possible, de façon à pouvoir obtenir un greffage optimal. Dans ces conditions, ce type de procédé peut conduire à l'obtention de particules de taille réduite et où les particules peuvent avoir un PIE qui peut atteindre des valeurs de l'ordre de 9,5, ce qui permet d'envisager leur mise en oeuvre jusqu'à un pH de l'ordre de 7,5.

Or, contre toute attente, les inventeurs ont maintenant découvert qu'il est possible de réaliser un greffage de particules à base d'un oxyde de fer menant à l'obtention de particules de taille réduite, sans avoir pour cela à mettre en oeuvre la sonication poussée enseignée par l'état de la technique, sous réserve de réaliser ce greffage sur des particules à base d'un oxyde de fer sous forme d'une dispersion colloïdale stable de particules, et en réalisant le traitement de surface dans les conditions de stabilité de la dispersion colloïdale (à savoir en évitant la floculation ou l'agrégation des particules). Dans ce cadre, les inventeurs ont mis en évidence que le diamètre hydrodynamique des particules greffées obtenues est sensiblement égal (voire identique, le plus souvent) au diamètre hydrodynamique des particules de la dispersion colloïdale de départ.

De façon encore plus surprenante, les travaux des inventeurs ont en outre permis d'établir que le greffage réalisé sur des particules à base d'oxyde de fer maintenues sous forme d'une dispersion colloïdale au cours du greffage permet d'obtenir des particules ayant un point isoélectrique supérieur à celui des particules obtenues par les procédés actuellement connus qui mettent en oeuvre un greffage sous sonication. A ce sujet, sans vouloir être lié par une théorie particulière, il semble que, de façon tout à fait inattendue, la mise en oeuvre d'une dispersion colloïdale stable en tant que telle, plutôt qu'une dispersion de particules maintenues en dispersion sous sonication, conduit à un greffage plus homogène des particules, ce qui rend les particules obtenues plus stables en milieu neutre.

De façon encore plus inattendue, les inventeurs ont également découvert au cours de leurs travaux que le greffage de composés tels que des aminosilanes sur les particules d'oxyde d'un ferrofluide améliore de façon sensible la stabilité thermique des particules, et ce notamment dans le cas où les particules sont des particules de maghémite, ce qui peut notamment se révéler intéressant pour leur utilisation dans la réalisation de matériaux composites ou des céramiques possédant des propriétés magnétiques à haute température.

Il s'avère en outre que la modification de surface telle que mise en oeuvre par les inventeurs permet d'obtenir des particules greffées en surface par des groupements aminés, par l'intermédiaire desquels des espèces chimiques, notamment des macromolécules, peuvent être immobilisées à la surface des particules greffées. En particulier, la modification de surface réalisée par les inventeurs permet l'immobilisation de molécules de polysaccharides, et en particulier de molécules de dextrane, par le biais de l'établissement de liaisons covalentes entre les polysaccharides et les groupements aminés.

Dans ce cadre spécifique, les travaux des inventeurs ont permis de mettre en évidence que, de façon surprenante, le greffage de macromolécules telles que des polysaccharides sur les groupements aminés peut être effectué en conservant une population de particules essentiellement individualisées, et en conservant en outre un diamètre hydrodynamique faible, typiquement inférieur à 50 nm, et le plus souvent inférieur à 40 nm.

Cette possibilité d'obtenir des particules magnétiques qui (i) possèdent un diamètre hydrodynamique réduit et (ii) sont greffées en surface par des macromolécules de type polysaccharides (dextrane notamment) qui sont liées de façon covalente, présente un grand intérêt, notamment pour la réalisation de compositions pour une administration chez l'animal ou l'être humain, et en particulier pour la réalisation de compositions d'agent de contraste pour l'imagerie par résonance magnétique (IRM).

En effet, les dispersions de particules ainsi obtenues constituent une alternative particulièrement intéressante aux dispersions de particules magnétiques actuellement utilisées notamment à titre d'agents de contraste pour l'IRM *in vivo.*

A l'heure actuelle, les dispersions de particules magnétiques destinées à une administration *in vivo* sont en effet des particules présentant un diamètre hydrodynamique le plus faible possible, typiquement inférieur à 100 nm, (notamment pour assurer une bonne diffusion tissulaire), et dont la surface est recouverte par des macromolécules hydrophiles telles que le dextrane généralement adsorbées sur la surface. Cet habillage de la surface des particules est notamment effectué pour rendre la surface des particules hydrophile et électriquement neutre, de façon à retarder la détection des particules par le système immunitaire (c'est à dire pour augmenter la demi-vie des particules *in vivo,* et retarder leur élimination par exemple au niveau du foie).

Ces particules habillées par des molécules de type dextrane de l'état de la technique ont, en général, une taille relativement importante, notamment due au fait que l'habillage de dextrane est effectué dans des conditions menant le plus souvent à une agglomération de plusieurs particules magnétiques, entourées par une couche de dextrane. On parvient toutefois à réaliser, dans certaines conditions de synthèse, telles que celles décrites par exemple dans US 4,452,773, des particules de taille relativement faible, typiquement de l'ordre de 50 nm, qui sont généralement désignées par le terme de *"USPIO"* (pour l'anglais *"Ultrasmall SuperParamagnetic Iron Oxides*") ou encore de "*MION*" (pour l'anglais "Microcrystalline Iron Oxide Nanoparticles"). Toutefois, quelle que soit leur taille, les particules magnétiques habillées de macromolécules de type dextrane actuellement connues mènent le plus souvent à des phénomènes de déplétion du dextrane lorsqu'elles sont administrées *in vivo,* ce qui réduit leur temps de séjour dans les organismes où elles sont introduites.

Au contraire, les dispersions de particules magnétiques greffées en surface par des macromolécules de type dextrane qui ont été mises au point par les inventeurs ne présentent pas de tels inconvénients, dans la mesure où les macromolécules de type dextrane sont liées de façon covalente à la surface de ces particules, ce qui permet d'éviter les phénomènes de déplétion observés avec les ferrofluides de type *"USPIO"* ou "*MION*" précités.

En outre, les travaux effectués par les inventeurs ont permis de mettre en évidence que, contrairement aux particules des ferrofluides de type *"USPIO"* ou "*MION*", les particules de surface modifiées par des macromolécules de type polysaccharides liés de façon covalente qui ont été,découvertes par les inventeurs peuvent elles-mêmes être fonctionnalisées, par exemple par des molécules d'intérêt biologique (par exemple des molécules d'effecteurs pour le ciblage vers des cellules ou des organes spécifiques, ou bien encore des principes actifs ou des oligonucléotides), et ce, sans augmenter de façon conséquente le diamètre hydrodynamique des particules, et en conservant en outre, le plus souvent, une population de particules essentiellement individualisées.

Sur la base de ces découvertes, la présente invention a notamment pour but de fournir des ferrofluides aqueux, présentant une très faible taille de particules, et qui soient stables en milieu neutre, et en particulier en milieu physiologique. De façon plus spécifique, l'invention a tout particulièrement pour but de fournir des ferrofluides stables jusqu'à un pH de 8, et notamment stable dans la gamme de pH allant de 6 à 8.

De façon générale, la présente invention vise également à fournir un procédé permettant de déplacer le domaine de stabilité d'un ferrofluide colloïdal dans une gamme de pH allant jusqu'à 8, englobant le pH des milieux physiologiques, sans modifier les propriétés magnétorhéologiques de ce ferrofluide, et notamment en conservant essentiellement ses propriétés magnétiques et le diamètre hydrodynamique des particules.

Un autre objectif de l'invention est de fournir des ferrofluides aqueux, présentant un faible taux d'agglomération interparticulaire en milieu neutre, et dans lesquels les particules peuvent être greffées par des espèces chimiques, telles que des macromolécules, sans conduire à des phénomènes d'agrégation interparticulaire. Dans ce cadre, l'invention se fixe plus spécifiquement pour but de fournir des ferrofluides comprenant des particules modifiées en surface par des molécules hydrophiles de type polysaccharide, notamment par des molécules de dextrane, administrable *in vivo,* et dont les particules ont un temps de séjour élevé au sein des organismes où elles sont introduites.

L'invention se fixe également pour but de fournir des dispersions de particules magnétiques de faible diamètre hydrodynamique, administrables *in vivo,* notamment par voie d'injection, adaptées pour permettre un ciblage vers des cellules ou des organes spécifiques, à des fins diagnostiques (composition d'agents de contraste biospécifiques pour IRM, notamment) ou à des fins thérapeutiques (vectorisation ciblée de principes actifs, en particulier de médicaments, par exemple).

Ainsi, selon un premier aspect, la présente invention a pour objet une dispersion aqueuse, désignée ci-après par "*ferrofluide aminé*", comprenant des particules (p) à base d'un oxyde de fer magnétique, de dimensions inférieures ou égale à 20 nm, dont la surface est modifiée par le greffage de groupements aminés R, liés de façon covalente à la surface desdites particules, dans laquelle le point isoélectrique des particules de surface ainsi modifiée est supérieur ou égal à 10.

Par "*dispersion aqueuse de particules*", on entend, au sens de la présente description, une dispersion de particules au sein d'un milieu de nature aqueuse, telle que l'eau, une solution aqueuse, ou bien encore un mélange eau/alcool.

Le ferrofluide aminé de l'invention présente, le plus souvent, des caractéristiques magnétiques et rhéologiques similaires à celles des ferrofluides aqueux de l'état de la technique qui sont constitués exclusivement de particules à base d'oxyde de fer dispersées au sein d'un milieu aqueux (ferrofluides "non greffés"). Ainsi, la taille et la morphologie des particules présentes sont en général similaires, ainsi que les propriétés du fluide sous sollicitation magnétique. Toutefois, à la différence des ferrofluides de type "non greffés", les ferrofluides aminés de l'invention présentent en outre l'avantage de posséder un point isoélectrique supérieur à 10, ce point isoélectrique étant le plus souvent supérieur où égal à 10,1, et avantageusement supérieur ou égal à 10,2, voire supérieur à 10,3, ce point isoélectrique étant en général inférieur ou égal à 11, et le plus souvent inférieur ou égal à 10,5. Ainsi, les particules (p) de surface modifiée présentes dans un ferrofluide selon l'invention sont en général stables vis-à-vis de l'agglomération interparticulaire dans des milieux ayant un pH inférieur à 8, et donc en particulier en milieu neutre, et notamment à des pH compris entre 6 et 8, par exemple en milieu physiologique, domaine où les ferrofluides "non greffés" présentent une nette tendance à la floculation.

De préférence, un ferrofluide aminé selon l'invention se présente sous la forme d'une dispersion aqueuse ayant un pH inférieur ou égal à 8. Le cas échéant, le ferrofluide aminé de l'invention se présente le plus souvent sous la forme d'une dispersion de particules, ayant un diamètre hydrodynamique moyen inférieur ou égal à 20 nm. En particulier, lorsque les particules (p) sont de dimensions suffisamment faibles, typiquement entre 5 et 7,5 nm, un ferrofluide aminé selon l'invention de pH inférieur ou égal à 8 se présente en général sous la forme d'une dispersion colloïdale stable de particules essentiellement individualisées.

Ainsi, le plus souvent, dans un ferrofluide aminé selon l'invention, au moins 95% des particules présentes, et de préférence au moins de 98% des particules présentes sont des particules individualisées, c'est à dire non agglomérées avec une ou plusieurs autres particules. En d'autres termes, un ferrofluide aminé selon l'invention présente en général un taux extrêmement réduit d'agrégats interparticulaires. Ainsi, en général, dans un ferrofluide aminé selon l'invention, au plus 5 (et de préférence au plus 2), éléments solides en suspension sur 100 sont sous forme d'agrégats de particules. En général, dans un ferrofluide aminé selon l'invention, on n'observe pas d'agrégats interparticulaires de taille supérieure à 30 nm.

Le "*diamètre hydrodynamique moyen*" auquel il est fait référence ici est le diamètre hydrodynamique moyen en nombre tel que mesuré par spectroscopie de corrélation de photons, par exemple par un appareil de type Zetasizer, tel que, notamment le Zetasizer 3000 HS commercialisé par Malvern Instruments. Cette méthode de mesure met en général en oeuvre une électrophorèse capillaire associée, à une analyse par interférométrie laser à effet Doppler. Des photographies réalisées en microscopie électronique à transmission confirment en général ce diamètre hydrodynamique moyen, de même que des mesures réalisées en diffraction de rayons X. En général, le diamètre hydrodynamique moyen des particules greffées dans les dispersions selon l'invention est compris entre 3 et 15 nm. Il peut être avantageux, notamment lorsqu'on envisage une utilisation des ferrofluides aminés de l'invention pour la préparation de composition pour une administration *in vivo,* en particulier par injection, que ce diamètre hydrodynamique soit inférieur à 12 nm, avantageusement inférieur à 10 nm, et plus préférentiellement à 8 nm.

Les particules (p) à base d'un oxyde de fer magnétique qui sont présentes au sein d'une composition selon l'invention, sont, de façon générale, des particules constituées, en tout ou partie, par un oxyde de fer présentant des propriétés magnétiques, et de préférence des propriétés ferrimagnétiques. Dans ces particules, la quantité d'oxyde de fer magnétique représente de préférence au moins 50% en masse, avantageusement au moins 60% en masse et encore plus préférentiellement au moins 90% en masse de la masse totale des composés inorganiques présents dans les particules, ces particules étant de préférence essentiellement constituées par un ou plusieurs oxyde(s) de fer, et l'oxyde de fer étant, dans tous les cas présent au moins au niveau de la surface des particule (p). De préférence, l'oxyde de fer magnétique constitutif des particules (p) de la dispersion aqueuse de l'invention est choisi parmi les ferrites de formule MFe₂O₄ (avec M = CO, Ni, Cd, Mn, Zn ou Mg), la magnétite Fe₃O₄, la maghémite (Fe₂O₃-γ), ou les mélanges de ces oxydes.

Selon un mode de réalisation préféré de l'invention, les particules (p) sont essentiellement constituées par de la magnétite (Fe₃O₄) et/ou de la maghémite (Fe₂O₃-γ), et avantageusement elles sont essentiellement constituées de maghémite.

Ainsi, on préfère que les particules (p) de l'invention soient constituées au moins à raison de 95% en masse et avantageusement au moins à raison de 98% en masse d'une ferrite, d'une maghémite et/ ou d'une magnétite.

Quelle que soit leur nature exacte, on préfère que les particules (p) soient des particules de nature cristalline. Avantageusement, les particules (p) sont au moins en partie (et de préférence pour l'essentiel) des monocristaux d'oxyde de fer, avantageusement des monocristaux de magnétite ou de maghémite.

Par ailleurs, de façon caractéristique, dans une dispersion aqueuse de particules magnétiques selon l'invention, les particules (p) sont greffées en surface par des groupements aminés R, chacun de ces groupements R comportant en général un groupement amine primaire -NH₂. Le plus souvent, ces groupements se présentent le plus souvent sous une forme protonée. Ces groupements aminés R, liés de façon covalente à la particule, sont avantageusement des groupements de formule -(A)-NH₂, dans laquelle le groupement -(A)- désigne une chaîne hydrocarbonée, comportant de 1 à 12 et de préférence au plus 8 atomes de carbone, cette chaîne étant éventuellement interrompue par un ou plusieurs groupements -NH-, généralement par 1 à 3 groupements -NH- le cas échéant. Le plus souvent, dans le groupement R, on préfère que le groupement -(A)- désigne une chaîne linéaire. Par ailleurs, le groupement -(A)- est avantageusement une chaîne hydrocarbonée saturée. Selon une variante envisageable, le groupement -(A)- peut toutefois comprendre un noyau aromatique.

De façon particulièrement avantageuse, les groupements aminés R liés de façon covalente à la surface des particules (p) d'une dispersion selon l'invention sont choisis parmi :
(i) les groupements de formule -(CH₂)n₁-NH₂, où n₁ =1, 2, 3, 4, 5, 6, 7 ou 8, n₁ étant de préférence égal à 3, 4, 5 ou 6, et avantageusement égal à 3 ;
(ii) les groupements de formule -(CH₂)n₂-NH-(CH₂)n_{2'} -NH₂, où n₂ et n₂' sont identiques ou différents et désignent chacun 1, 2, 3, 4, 5 ou 6, de préférence 1, 2 ou 3 et avantageusement 2, étant entendu que (n₂ + n_{2'}) reste compris entre 2 et 9;
(iii) les groupements de formule -(CH₂)n₃-NH-(CH₂)n_{3'} -NH-(CH₂)n_{3"}-NH₂, où n₃, n_{3'} et n_{3"} sont identiques ou différents et désignent chacun 1, 2, 3, ou 4, de préférence 1, 2 ou 3, et avantageusement 2, étant entendu que (n₃+ n_{3'} + n_{3"}) reste compris entre 3 et 12.

A titre d'exemple, le groupement R peut représenter un groupement choisi parmi les groupements suivants :
-(CH₂)₃-NH₂;
-(CH₂)₄-NH₂;
- (CH₂)₃-NH-(CH₂)₂-NH₂.
- (CH₂)₃-NH-(CH₂)₆-NH₂.
- (CH₂)₃-NH-CH(CH)₃-CH₂-NH₂.
- (CH₂)₃-NH-(CH₂)₂-NH-(CH₂)₂-NH₂ ; 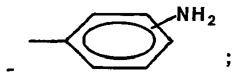 ou

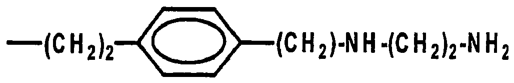

Quelle que soit leur nature exacte, les groupements aminés R sont, de façon caractéristique, des groupements liés aux particules (p) par l'intermédiaire d'une liaison covalente. Le plus souvent, cette liaison covalente est assurée par l'intermédiaire d'un atome de silicium, selon la structure schématique suivante :

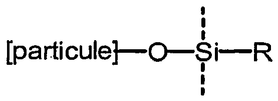

En d'autres termes, les groupements R présents à la surface des particules (p) sont généralement obtenus par réaction d'un silane porteur du groupement R sur les particules (p) à base d'oxyde de fer magnétique.

En général, la quantité moyenne de groupements R liés de façon covalente à la surface des particules (p) est au moins égale à 2 micromoles par m² (µmol/m²) et elle est le plus souvent comprise entre 3 et 10 µmol/m², étant entendu qu'il est préférable qu'elle reste supérieure à 4 µmol/m². En général cette quantité reste inférieure à 8 µmol/m².

Il est à souligner qu'une dispersion selon l'invention peut comprendre des particules greffées en nombre relativement important. Ainsi, on peut envisager pour une dispersion selon l'invention, des concentrations en particules (p) supérieures à 30 grammes d'oxyde de fer par litre, voire supérieures à 100 g/l et même dans certains cas supérieures à 500 g/l. Ainsi, typiquement, un ferrofluide aminé selon l'invention peut comprendre des particules (p) à raison de 50 à 600 g d'oxyde de fer. Compte tenu de la possibilité de mettre en oeuvre de telles concentrations, les dispersions selon l'invention peuvent notamment se révéler particulièrement utiles à titre de ferrofluides à comportement superparamagnétique. Le cas échéant, les particules (p) sont de préférence des particules monocristallines, de préférence des particules "monodomaine" de maghémite.

Selon un autre aspect, la présente invention concerne un procédé de préparation des ferrofluides aminés tels que décrits précédemment.

Ce procédé est caractérisé en ce qu'il comprend les étapes consistant à :
(A) fournir une dispersion aqueuse acide de particules (p₀) à base d'un oxyde de fer magnétique de dimensions inférieures à 20 nm, ladite dispersion présentant, en milieu acide, une stabilité colloïdale au moins dans une gamme de pH, cette stabilité étant telle que, dans ladite gamme de pH, on observe, sans avoir à maintenir une agitation, une dispersion de particules essentiellement individualisées ayant un diamètre hydrodynamique moyen inférieur à 20 nm ;
(B) mettre en contact la dispersion colloïdale acide de l'étape (A) avec des silanes de formule R-SiX₁X₂X₃, dans laquelle :
   - R désigne un groupement aminé tel que défini précédemment ;
   - X₁, X₂, et X₃ sont des groupements identiques ou différents, désignant chacun un groupe hydrolysable en milieu acide,
   cette mise en contact étant effectuée en maintenant le milieu dans la gamme de pH où la stabilité colloïdale de la dispersion est assurée, ce par quoi on obtient, par hydrolyse des groupements X₁, X₂, et X₃, la formation de silanols dans le milieu de dispersion des particules ;
(C) ajouter au milieu réactionnel un agent mouillant hydrosoluble de température d'ébullition supérieure à celle de l'eau, puis chauffer le milieu réactionnel à une température suffisante pour éliminer l'eau mais sans éliminer l'agent mouillant, ce par quoi on obtient une condensation des silanols à la surface des particules, en évitant l'agglomération des particules (qui restent dispersées dans l'agent mouillant) ; et
(D) récupérer les particules obtenues à l'issue de l'étape (C) et les disperser en milieu aqueux, ce par quoi on obtient un ferrofluide aminé selon l'invention.

On constate en pratique que le diamètre hydrodynamique des particules présentes et les propriétés magnétiques des ferrofluides aminés obtenues à l'issue de l'étape (D) sont extrêmement similaires, sinon identiques, à celles de la dispersion aqueuse colloïdale acide fournie dans l'étape (A). Ainsi, les propriétés magnétorhéologiques de la dispersion obtenue à l'issue du procédé de préparation de l'invention sont en général entièrement déterminées par celles des dispersions aqueuses colloïdales acides mises en oeuvre dans l'étape (A).

Les dispersions aqueuses colloïdales acides mises en oeuvre dans l'étape (A) sont des dispersions aqueuses colloïdales de particules à base d'oxyde de fer de pH généralement inférieur à 5, le plus souvent de pH compris entre 2 et 4, et avantageusement inférieur à 3. De préférence, la dispersion aqueuse acide colloïdale de l'étape (A), est telle que, dans la gamme de pH où la stabilité colloïdale est assurée, le diamètre hydrodynamique moyen des particules observées en suspension est compris entre 3 et 15 nm, ce diamètre pouvant dans certains cas être inférieur ou égal à 12 nm, voire inférieur ou égal à 10 nm, ou bien, dans certains cas particuliers, inférieur ou égal à 8 nm.

Par ailleurs, on préfère que, dans la gamme de pH où la stabilité colloïdale est assurée, moins de 5 % en nombre, avantageusement moins de 2% , et de préférence moins de 1 % en nombre des espèces solides observées en suspension soient des agglomérats de plusieurs particules.

De façon générale, la dispersion aqueuse acide de l'étape (A) peut ainsi être choisie parmi la plupart des "*ferrofluides acides*" aqueux stabilisés connus de l'état de la technique présentant un diamètre hydrodynamique et une stabilité colloïdale adaptée. Ainsi, la dispersion colloïdale acide de l'étape (A) peut avantageusement être un ferrofluide aqueux acide de particules de ferrites, de magnétite et/ou de maghémite tel qu'obtenu en mettant en oeuvre le procédé décrite par Massart et al., par exemple dans le brevet US 4,329,241 ou dans IEEE Trans. Magn., MAG-17(2), pp. 1247-1248 (1981*).*

De façon particulièrement avantageuse, la dispersion aqueuse colloïdale acide de l'étape (A) est une dispersion de particules de maghémite, préparée selon un procédé comprenant les étapes consistant à :
(a1) réaliser une co-précipitation de sels ferreux et ferriques, en additionnant une base, de préférence en excès, à une solution aqueuse contenant un mélange de sels de fer (II) (et de sels de fer (III), de façon à obtenir un floculat de particules de magnétite ;
(a2) après une éventuelle séparation du floculat, par exemple par décantation magnétique, (ce qui se révèle en général avantageux), traiter ledit floculat de particules obtenu avec un acide choisi parmi HNO₃, HCl ou CH₃COOH, et avantageusement par HNO₃, ce qui permet d'acidifier la surface des particules et de réaliser une oxydation superficielle des particules par solubilisation d'ions ferreux ;
(a3) ajouter au milieu une solution d'un sel ferrique, et laisser réagir, de préférence en chauffant le milieu (avantageusement, la réaction est conduite en portant le milieu à ébullition, ce par quoi on obtient une oxydation des particules sous la forme de particules de maghémite) ; et
(a4) disperser le floculat de particules de maghémite obtenu à l'issue de l'étape précédente en milieu aqueux, par ajout d'un acide choisi parmi HNO₃ ou HClO₄.

Le cas échéant, les conditions mises en oeuvre dans l'étape (a1) du procédé ci-dessus sont de préférence les suivantes :
- sels de Fer II utilisé : le chlorure ferreux ou le sulfate ferreux, et avantageusement le chlorure ferreux ;
- sels de Fer III utilisé : le chlorure ferrique ou le nitrate ferrique, et avantageusement le chlorure ferrique ;
- ratio molaire Fe(III)/Fe(II) initial : de préférence de l'ordre de 2 ;
- base utilisée pour la co-précipitation : l'ammoniaque ou la soude.

Lorsque de la soude est utilisée, il est préférable que le milieu de co-précipitation contienne du nitrate de sodium.

De façon plus générale, il est à noter que, le plus souvent, le traitement effectué dans les étapes (B) à (D) du procédé de l'invention est un traitement de surface qui n'affecte pas la nature physico-chimique interne des particules de la dispersion aqueuse colloïdale acide de l'étape (A). Par conséquent, la nature chimique des particules de la dispersion de l'étape (A) est en général la même que celles des particules (p) de la dispersion qu'on souhaite obtenir à l'issue de l'étape (D).

Selon un mode de réalisation qui peut se révéler avantageux, les particules présentes dans les dispersions colloïdales acides de l'étape (A) présentent une surface spécifique BET comprise entre 50 m²/g et 1000 m²/g, cette surface spécifique étant de préférence comprise entre 100 et 200 m²/g et avantageusement de l'ordre de 130 m²/g.

L'étape (B) du procédé de l'invention consiste à mettre en contact la dispersion colloïdale de l'étape (A) avec des silanes présentant un groupement R aminé, en maintenant les particules à l'état dispersé. Cette étape de mise en contact des silanes et des particules à l'état dispersé est en général réalisée par addition de silanes dans la suspension de particule fournie lors de l'étape (A), et on laisse en général réagir le milieu obtenu pendant une durée de 2 à 15 heures , le plus souvent sous agitation. En général, la réaction est conduite à température ambiante.

Les silanes mis en oeuvre dans l'étape (B) sont de préférence des trialcoxysilanes aminés de formule R-Si(-OR')(-OR")(-OR"'), dans laquelle :
- R est un groupe aminé tel que défini précédemment ; et
- R', R" et R"', identiques ou différents, désignent chacun un groupement alkyle comportant de 1 à 5 atomes de carbone, chacun de R', R" et R"' désignant de préférence un groupement méthyle ou un groupement éthyle, et avantageusement un groupement méthyle.

Ainsi, à titre de silanes particulièrement avantageux dans l'étape (B), ont peut citer notamment :
- le γ-aminopropyltriméthoxysilane (CH₃O)₃-Si-(CH₂)₃-NH₂ ;
- le N-β-aminoéthyl-γ-aminopropyltriméthoxysilane (CH₃O)₃-Si-(CH₂)₃-NH-(CH₂)₂-NH₂ ;
- le N'-β-aminoéthyl-N-β-aminoéthyl-γ-aminopropyltriméthoxysilane (CH₃O)₃-Si-(CH₂)₃-NH-(CH₂)₂-NH-(CH₂)₂-NH₂.

D'autres silanes envisageables dans l'étape (B) sont :
- le 4-aminobutyltriéthoxysilane,
- le N-(2-aminoéthyl)-3-aminoisobutyl-méthyldiméthoxysilane,
- l'aminoéthylaminométhyl)phénéthyl-triméthoxysilane,
- le N-(2-aminoéthyl)-3-aminopropylinéthyl-diméthoxysilane,
- le N-(2-aminoéthyl)-3-aminopropyltriméthoxysilane,
- le N-(6-aminohexyl)aminopropyl-triméthoxysilane,
- le m-aminophényltriméthoxysilane,
- le p-aminophényltriméthoxysilane,
- le 3-aminopropylméthylbis(triméthylsiloxy)silane,
- le 3-aminopropylméthyldiéthoxysilane,
- le 3-aminopropyltriéthoxysilane,
- le 3-aminopropyltriméthoxysilane.

En général, quelle que soit leur nature, les silanes de l'étape (B) sont introduits dans le milieu de l'étape (A) sous forme pure, ou sous la forme d'une solution dans un solvant organique, de préférence, le cas échéant, dans un alcool tel que le méthanol ou l'éthanol et plus avantageusement dans le méthanol. Lorsque les silanes sont introduits en solution, la concentration en silane dans le solvant organique est avantageusement comprise entre 3 et 4% en poids.

La quantité de silane introduite dans l'étape (B) est en général ajustée pour obtenir une couverture la plus complète possible de la surface des particules par les groupements silanes A cet effet, on préfère en général que la quantité de silanes porteurs des groupements R introduite, rapportée à la surface totale disponible développée par les particules (p) de la dispersion colloïdale acide de l'étape (A) soit au moins égale à 2,5 µmol/m², et de préférence au moins égale à 10 µmol/m². La surface totale disponible développée par les particules (p) est calculée en multipliant la masse des particules (en g) par la surface spécifique BET de ces particules (en m²/g).

De façon caractéristique, dans le procédé de l'invention, l'étape (B) est suivie par une étape (C) de déshydratation, effectuée en présence d'un agent mouillant. Ce traitement a pour but d'effectuer la déshydratation en laissant les particules en suspension dans l'agent mouillant, ce par quoi on obtient un greffage efficace des espèces silanols à la surface des particules d'oxyde, en évitant des phénomènes d'agrégation interparticulaire qui seraient observés en l'absence d'agent mouillant. Cette étape de traitement thermique peut être notamment du type de celle mise en oeuvre dans les procédés décrits dans les brevets US 4, 524 ,088 ou US 4 ,695, 393.

Lorsque les silanes de l'étape (B) sont introduits en solution dans un solvant organique, l'agent mouillant de l'étape (C) est de préférence un agent qui est soluble dans le solvant organique solubilisant les silanes introduits dans l'étape (B) et qui possède une température d'ébullition supérieure à celle dudit solvant organique, et l'étape (C) comprend en général une étape de chauffage à une température suffisante pour éliminer ledit solvant organique sans éliminer l'agent mouillant. Cette élimination du solvant peut être réalisée conjointement à l'élimination de l'eau, ou bien de façon séparée, et de préférence préalablement à l'élimination de l'eau, le cas échéant.

Dans tous les cas de figure, un agent mouillant particulièrement intéressant pour la mise en oeuvre de l'étape (C) est le glycérol.

De préférence, quelle que soit la nature de l'agent mouillant, le chauffage de l'étape (C) est conduit sous vide, ce qui permet notamment de travailler à température modérée et de protéger la couche de greffage en cours de synthèse. Ainsi, lorsque le chauffage de l'étape (C) est conduit sous vide, il s'avère particulièrement avantageux de conduire la déshydratation de l'étape (C) à une température inférieure ou égale à 130°C, et de préférence inférieure à 120° C (typiquement de l'ordre de 80 à 100° C). Par ailleurs, si l'étape (C) comprend une étape d'élimination d'un solvant organique, cette étape est de préférence réalisée à une température la plus basse possible, et avantageusement à une température inférieure ou égale à 50° C.

Suite à l'étape (C), on obtient des particules de surface modifiée par les groupements R au sein d'un milieu essentiellement constitué de l'agent mouillant (du glycérol le plus souvent) et qui contient en général également des silanes introduits lors de l'étape (B), qui n'ont pas réagi, et d'éventuels autres sous-produits. L'étape (D) du procédé consiste à récupérer les particules de ce milieu puis à les disperser en milieu aqueux.

Le plus souvent, l'étape (D) comprend un lavage des particules obtenues à l'issu de l'étape (C), par exemple par de l'acétone ou par un mélange eau/ acétone. Le cas échéant, on prend soin de ne pas laisser sécher les particules au cours du lavage, ce qui permet notamment d'éviter tout phénomène d'agglomération interparticulaire. Le floculat de particules non séché obtenu à l'issue du lavage est ensuite dispersé en milieu aqueux. L'absence de séchage lors du lavage permet une dispersion optimale des particules en milieu aqueux. Les traces de solvant de lavage introduites dans le milieu aqueux (notamment les traces d'acétone) peuvent ensuite être éliminées, par exemple par entraînement sous vide.

Avantageusement, la dispersion des particules effectuée lors de l'étape (D) est réalisée en plaçant les particules récupérées à l'issue de l'étape (C) dans de l'eau et en diminuant progressivement le pH du milieu par addition lente d'un acide tel que l'acide nitrique, l'acide chlorhydrique ou l'acide perchlorique. De préférence on utilise l'acide nitrique. Par ailleurs, la diminution progressive du pH s'effectue typiquement en plusieurs étapes, par décrémentation du pH du milieu d'une unité de pH à chaque étape, de préférence sous agitation modérée. Cette étape de diminution progressive du pH, dite de "*peptisation par un acide*", permet de particulièrement bien préserver la couche de greffage réalisée à la surface des particules. Lorsqu'on utilise la technique de peptisation précitée, on observe en général une dispersion optimale du floculat introduit sous la forme de particules individualisées lorsqu'on atteint un pH de l'ordre de 3. Une fois cette dispersion réalisée, le pH peut être modifié dans la gamme de valeurs de pH inférieures à 8 sans affecter la stabilité de la suspension obtenue. En particulier, on peut modifier le pH par addition d'une base, par exemple la soude ou l'ammoniaque, notamment pour obtenir un ferrofluide aminé stable de pH compris entre 6 et 8.

Selon un autre aspect, la présente invention a pour objet les utilisations envisageables pour les ferrofluides aminés définis précédemment.

Comme on l'a souligné, les ferrofluides aminés de l'invention présentent des caractéristiques similaires à celles des ferrofluides usuels (taille réduite de particules notamment), mais ils présentent en outre l'avantage d'être stables en milieu neutre, et en particulier dans la gamme de pH comprise entre 6 et 8. De façon générale, les ferrofluides aminés de l'invention peuvent donc être utilisés dans tous les domaines d'application connus des ferrofluides classiques, mais leur stabilité en milieu neutre les rend adaptés à d'autres type d'applications.

Ainsi, compte tenu de leur stabilité, notamment à des pH de l'ordre de 7 et en particulier en milieu physiologique, les ferrofluides aminés de l'invention se révèlent en particulier utiles pour la préparation de composition d'agents de contraste pour l'imagerie par résonance magnétique, tout particulièrement *in vivo.* Dans ce type d'application, la faible taille des particules modifiées par les groupements aminés R s'avère particulièrement adaptée à une bonne diffusion tissulaire. De plus, les inventeurs ont d'ailleurs mis en évidence que la modification de surface par les groupements aminés R, effectuée dans les conditions des étapes (A) et (B), permet également d'augmenter la stabilité des ferrofluides acides initiaux vis-à-vis de la présence d'anions, tels que les anions chlorure par exemple, qui sont connus comme capables d'induire une perte de la stabilité des ferrofluides acides, par compensation des charges positives de surface des particules. Cette stabilisation en présence d'anions se révèle particulièrement avantageuse pour des applications *in vivo* où le ferrofluide est souvent mis en contact avec des milieux salins.

Ainsi, de façon générale, les ferrofluides aminés de l'invention se révèlent particulièrement intéressants pour la préparation de compositions, notamment à usage thérapeutique et/ou diagnostique, administrables par voie orale ou parentérale chez l'homme ou chez l'animal, et notamment pour la préparation de compositions injectables. Les compositions pour une administration chez l'homme ou l'animal obtenues dans ce cadre, et notamment les compositions injectables d'agents de contraste pour l'imagerie par résonance magnétique, constituent un autre objet de l'invention.

La stabilité des ferrofluides aminés de l'invention sur toute la gamme de pH inférieur à 8 permet en outre d'envisager leur utilisation dans d'autres applications, et notamment à titre de charge magnétique dans différentes compositions ou matériaux magnétiques, par exemple dans des compostions ou des matériaux polymères (où les particules du ferrofluide peuvent par exemple assurer une réticulation entre plusieurs molécules de polymères), ou bien encore dans des matériaux de type céramique. Dans ce type d'application, les ferrofluides aminés de l'invention se révèlent en particulier utiles lorsque la préparation des compositions ou matériaux met en oeuvre une formulation aqueuse. En ce qui concerne ce type d'utilisation, il est à rappeler que les ferrofluides aminés de l'invention contiennent des particules qui sont stabilisées thermiquement. En particulier, elles sont stables à une température supérieure à 740 K, et en général jusqu'à 1050K. Ces particules peuvent être utilisées à titre de charges magnétiques pour la fabrication de matériaux magnétiques, en particulier des céramiques, qui peuvent être utilisés à des température supérieures à 740K, sans perte de leurs propriétés magnétiques.

Par ailleurs, comme on l'a souligné, les ferrofluides aminés de la présente invention contiennent des particules (p) sur lesquelles sont liés des groupements aminés R, qui peuvent réagir avec des espèces chimiques, de façon à modifier la chimie de surface des particules du ferrofluide aminé. De façon surprenante, les inventeurs ont découvert que la stabilité des particules des ferrofluides aminés de l'invention est telle que la modification de la surface des particules (p) par réaction d'espèces chimiques sur les groupements R peut être réalisée sans que soient observés de phénomènes d'agrégation interparticulaire. Ainsi, les ferrofluides aminés de l'invention peuvent être utilisés pour la préparation de suspensions de particules magnétiques essentiellement individualisées, à la surface desquelles sont immobilisées des espèces chimiques, où l'immobilisation des espèces chimiques est réalisée en établissant une liaison, de préférence covalente, entre lesdites espèces chimiques et les groupements aminés R présents à la surface des particules (p) contenus dans les ferrofluides aminés.

Dans ce cadre, la présente invention a plus spécifiquement pour objet un procédé de modification de la surface des particules (p) d'un ferrofluide aminé selon l'invention, ce procédé comprenant une étape (G1) consistant à faire réagir ledit ferrofluide aminé avec des espèces chimiques E susceptibles de former une liaison, de préférence covalente, avec les groupements aminés R présents à la surface des particules (p), cette réaction étant conduite dans les conditions de stabilité du ferrofluide aminé, à savoir, en général à un pH inférieur à 8, et préférence à un pH inférieur, avantageusement à un pH inférieur ou égal à 4, et de préférence à un pH inférieur ou égal à 3.

La nature des espèces E mise en oeuvre dans l'étape (G1) peut varier en une assez large mesure et il est des compétences de l'homme du métier de déterminer les espèces adaptées à la modification de surface de l'étape (G1), dans les conditions de stabilité du ferrofluide aminé utilisé.

Le plus souvent, lorsqu'on met en oeuvre l'étape (G1) précitée, on préfère que les groupements aminés R présents sur les particules (p) du ferrofluide aminé présentent des groupements -NH₂. Dans ce cas, les espèces chimiques E utilisées dans l'étape (G1) présentent de préférence des groupements aldéhyde - CHO, carboxyle -COO-, anhydride d'acide, isothiocyanate -SCN, cyanate -CN, ou maléimide.

Avantageusement, lorsque les groupements aminés R présentent des groupements -NH₂, les espèces chimiques E utilisées dans l'étape (G1) présentent des groupements aldéhyde. Dans ce cas, la réaction de l'étape (G1) consiste en général à faire réagir le ferrofluide aminé avec les espèces chimiques E porteuses de groupements -CHO, en présence d'un agent réducteur, tel que NaBH4 par exemple. Schématiquement, la réaction qui a lieu à la surface de la particule peut alors être représentée par la séquence réactionnelle suivante :
*Etape 1* : [*particule*]-*NH₂* + [*E*]-*CHO* → [*particule*]-*N*=*CH*-[*E*]
*Etape* 2 : [*particule*]-*N*=*CH*-[*E*] + [*agent réducteur*] → [*particule*]-*CH₂*-*NH*-[*E*].

Selon un mode de réalisation préférentiel, les espèces E mises en oeuvre dans l'étape (G1) sont des molécules de polysaccharides, et avantageusement des molécules de dextrane, dont une partie des groupements -OH a été oxydée en groupement -CHO. Le cas échéant, la modification partielle des groupements -OH en groupement -CHO est par exemple réalisée par une oxydation ménagée par un oxydant tel que le periodate de sodium NaIO₄ , notamment selon la méthode décrite par Molteni dans Methods in Enzymology, 112, 285-295, (1985).

On peut toutefois envisager l'utilisation d'autres espèces E dans l'étape (G1). Ainsi, les espèces E peuvent par exemple être des agents colorants, ce par quoi les dispersions obtenues peuvent par exemple être utilisées pour la réalisation de compositions d'encre magnétique. Selon une autre variante envisageable, les espèces E peuvent être des composés présentant une affinité pour un composé donné, auquel cas les dispersions obtenues pourront être utilisées pour réaliser une extraction magnétique dudit composé (réaction entre les particules et le composé, puis extraction des particules ayant réagi par un champ magnétique). Cette application pourra être mise à profit notamment dans des procédés de purification, de dépollution (en particulier d'eaux usées), ou d'extraction de composés d'intérêt.

Les dispersions aqueuses de particules à base d'un oxyde de fer magnétique de surface modifiée à la surface desquelles sont immobilisées des espèces chimiques E, qu'on peut obtenir selon le procédé précité de modification des ferrofluides aminés de l'invention, en mettant en oeuvre l'étape (G1), constituent un objet spécifique de la présente invention. Ces compositions seront désignées par le terme de "*ferrofluide aminé modifié*" dans la suite de la description.

Dans un "*ferrofluide aminé modifié*" selon l'invention, les particules en présence sont généralement bien individualisées. Ainsi, le plus souvent, au moins 90% en nombre , et de préférence au moins 95% en nombre des éléments solides contenus dans un ferrofluide aminé modifié selon l'invention sont des particules individualisées comprenant un noyau central unique, à base d'un oxyde de fer magnétique, ce noyau ayant des dimensions inférieures à 20 nm.

Le plus souvent, un ferrofluide aminé modifié selon l'invention présente une structure extrêmement similaire au ferrofluide aminé initial, avec uniquement une modification de la surface des particules. En particulier, il est à noter que l'étape (G1) ne modifie pas les particules à base d'oxyde de fer en elles-mêmes, qui sont généralement identiques à celles présentes dans le ferrofluide initial. Ainsi, dans un ferrofluide aminé modifié selon l'invention, les particules à base d'oxyde de fer sont de préférence des particules de maghémite, de préférences monocristallines ("monodomaine magnétique") qui ont avantageusement une taille inférieure à 20nm, et généralement comprise entre 3 et 15 nm.

Les ferrofluides aminés modifiés où les espèces E sont des polysaccharides dont une partie des groupements -OH a été oxydée en groupement -CHO constituent des ferrofluides aminés modifiés selon l'invention particulièrement avantageux. Sont tout particulièrement intéressants dans ce cadre les ferrofluides aminés modifiés où les espèces E sont des molécules de dextrane dont une partie des groupements -OH a été oxydée en groupement -CHO.

Les inventeurs ont mis en évidence que ces ferrofluides aminés modifiés particuliers présentent les avantages des ferrofluides de type *"USPIO"* (ou "*MION*") actuellement connus (à savoir une faible taille de particules et une couverture des particules par des polysaccharides) avec en outre un avantage supplémentaire, à savoir une immobilisation efficace des molécules de polysaccharides à la surface des particules. Cette immobilisation effective, qui n'est pas assurée dans les ferrofluides actuellement connus, permet (i) d'augmenter la demi-vie plasmatique des particules lorsqu'elles sont administrées *in vivo* (la demi -vie des particules, qui reflète la durée pendant laquelle les particules ne sont pas reconnues et éliminées par le système immunitaire, est en particulier dépendante de la stabilité de l'immobilisation des polysaccharides de type dextrane à la surface des particules); et (ii) d'autoriser un greffage d'espèces chimiques sur la couche de couverture de polysaccharides, sans conduire à des phénomènes de déplétion des saccharides ou d'agglomération interparticulaire.

Dans la suite de la description, les ferrofluides aminés modifiés où les espèces E sont des polysaccharides dont une partie des groupements -OH a été oxydée en groupements -CHO, qui constituent une alternative particulièrement avantageuse aux ferrofluides de type "USPIO", seront désignés par le terme de "(*ferrofluide VUSPIO*" (pour l'anglais "*Versatile* Ultrasmall SuperParamagnetic Iron Oxides"). Ces ferrofluides dits *"VUSPIO"* constituent un objet particulier de la présente invention.

Un ferrofluide *"VUSPIO"* se présente en général sous la forme d'une dispersion aqueuse de particules à base d'un oxyde de fer magnétique, de dimensions (généralement inférieures à 20 nm, et typiquement comprises entre 3 et 15 nm) à la surface desquelles sont immobilisées par liaison covalente des molécules de polysaccharide liées à la surface par l'intermédiaire de liaisons covalentes -NH-CH₂-. Sont particulièrement intéressants les ferrofluides *"VUSPIO"* où les molécules de polysaccharides sont des molécules de dextrane.

Le plus souvent, dans un ferrofluide *"VUSPIO",* le diamètre hydrodynamique moyen des particules de surface modifiée par les molécules de polysaccharide est inférieur à 50 nm, et il est en général inférieur ou égal à 40 nm, voire à 30 nm, en particulier lorsque les molécules de polysaccharides sont des molécules de dextrane.

Par ailleurs, il est possible, dans un ferrofluide "VUSPIO", d'obtenir un très faible taux d'agglomération particulaire extrêmement réduit. Ainsi, un ferrofluide "VUSPIO" peut par exemple être tel qu'à titre d'éléments solides en suspension, il contient pour l'essentiel (à savoir pour au moins 90%, de préférence pour au moins 95% et avantageusement pour au moins 98%) des particules individualisées comprenant un noyau central unique, à base d'un oxyde de fer, ayant des dimensions inférieures à 20 nm, ce noyau étant entouré par une couche comprenant les molécules de polysaccharide liées de façon covalente. Cette structure peut notamment être observée lorsque les polysaccharides sont des molécules de dextrane.

De façon plus générale, un ferrofluide "VUSPIO" peut comprendre des particules comprenant un "noyau" constitué de 1 à 250 particules inorganiques, "piégées" dans une matrice de polysaccharides. Le nombre de particules piégées dans la matrice de polysaccharide peut toutefois être modulé en une assez large mesure. Selon un mode particulièrement avantageux, ce nombre de noyaux est inférieur à 100, de préférence inférieur à 50, et avantageusement inférieur à 10. De façon particulièrement avantageuse, ce nombre est inférieur à 5 et il est particulièrement avantageux que ce nombre soit de l'ordre de 1 à 3. Les ferrofluides "VUSPIO" où l'essentiel des particules sont constituées d'un unique noyau entouré par une matrice de polysaccharides constituent un objet particulier de l'invention.

Dans un ferrofluide "VUSPIO" selon l'invention, le rapport massique oxyde de fer/polysaccharide est de préférence compris entre 10:90 et 90:10 et avantageusement compris entre 30:70 et 40:60, ce ratio étant avantageusement de l'ordre de 35:65.

De préférence, dans un ferrofluide "VUSPIO" selon l'invention, les particules à base d'oxyde de fer magnétique sont essentiellement constituées de maghémite (Fe₂O₃-γ). Avantageusement, ces particules sont monocristallines ("monodomaine magnétique"). En particulier dans ce cas, les ferrofluides "VUSPIO" selon l'invention se révèlent particulièrement utiles pour la préparation de compositions d'agents de contraste pour l'imagerie par résonance magnétique, et notamment pour la préparation de compositions pour une administration *in vivo,* en particulier par injection. Les compositions d'agents de contraste pour l'imagerie par résonance magnétique qui comprennent un ferrofluide *"VUSPIO"* constituent un autre objet particulier de la présente invention.

Par ailleurs, il est à noter que, dans un ferrofluide *"VUSPIO",* une partie des groupements -OH des molécules de polysaccharide qui sont immobilisées à la surface des particules sont oxydés sous la forme de groupements -CHO. En effet, le plus souvent, une partie seulement des groupements -CHO des polysaccharides oxydés utilisés dans l'étape (G1) de modification d'un ferrofluide aminé selon l'invention est engagée dans la réaction avec les groupements aminés R. Les groupements -CHO résiduels s'avèrent particulièrement avantageux, dans la mesure où ils permettent d'immobiliser des espèces chimiques à la surface des particules des ferrofluides *"VUSPIO".* De façon plus générale, que de tels groupements résiduels -CHO existent ou non, la stabilité des ferrofluides "*VUSPIO*" est le plus souvent telle qu'on peut envisager le greffage d'espèces chimiques à la surface des particules de ces ferrofluides spécifique sans observer de phénomènes notables d'agglomération interparticulaire. En d'autres termes, un ferrofluide "*VUSPIO*" se révèle utile pour la fabrication de dispersions de particules magnétiques individualisée à la surface desquelles sont immobilisées des espèces chimiques F, où le greffage des espèces chimiques est réalisé en établissant une liaison, de préférence covalente, entre lesdites espèces chimiques F et les molécules de polysaccharide.

Selon un dernier aspect, la présente invention a pour objet cette utilisation particulière des ferrofluides *"VUSPIO",* ainsi que les compositions obtenues par modification de la surface des particules de ces ferrofluides spécifiques.

En particulier, l'invention a pour objet l'utilisation des ferrofluides *"VUSPIO"* où les polysaccharides immobilisés ont une partie de leurs groupements OH oxydés sous la forme de groupements -CHO s'avèrent utiles pour la fabrication de dispersions de particules magnétiques individualisées, à la surface desquelles sont immobilisées des espèces chimiques F, où l'immobilisation desdites espèces F est réalisée en établissant une liaison covalente entre les espèces F et les groupements -CHO présents sur les molécules de polysaccharide. Le cas échéant, les espèces F présentent de préférence un ou plusieurs groupements NH₂.

Plus spécifiquement, l'invention a pour objet un procédé de modification de la surface des particules présentes dans un ferrofluide "VUSPIO", comprenant une étape (G2) consistant à faire réagir ledit ferrofluide "VUSPIO" avec des espèces chimiques F susceptibles de former une liaison , de préférence covalente, avec les molécules de polysaccharide. Dans ce procédé, le ferrofluide "VUSPIO" utilisé est de préférence un ferrofluide où les polysaccharides immobilisés ont une partie de leurs groupements OH oxydés sous la forme de groupements -CHO, les espèces chimiques F ayant alors, en général, un groupement -NH₂. Le cas échéant, l'étape (G2) consiste en général à faire réagir le ferrofluide "VUSPIO" avec les espèces F puis à traiter le milieu obtenu par un agent réducteur, par exemple le borohydrure de sodium.

En mettant en oeuvre le procédé de modification précité, on obtient des dispersions aqueuses de particules à base d'un oxyde de fer magnétique à la surface desquelles sont immobilisées par liaison covalente des molécules de polysaccharide, avantageusement des molécule de dextrane, par l'intermédiaire de liaisons covalente de formule -NH-CH₂-, ces molécules de polysaccharide étant elle-mêmes liées à des espèces chimiques F, de préférence également par l'intermédiaire de liaisons covalentes. Ces suspensions aqueuses particulières seront désignées dans la suite de la description par le terme de "*ferrofluide VUSPIO fonctionnalisé*".

Les ferrofluides "*VUSPIO fonctionnalisés*" selon l'invention peuvent être utilisées dans de nombreux domaine d'application.

Ainsi, ces ferrofluides peuvent notamment être utilisées pour la préparation de compositions d'agents de contraste pour imagerie médicale ayant une affinité pour des cellules, des tissus ou des organes donnés. Dans ce cadre, les espèces F sont choisies parmi des espèces présentant une affinité vis-à-vis des cellules, des tissus ou des organes pour lesquels la spécificité est recherchée. Les compositions d'agents de contraste pour imagerie médicale comprenant un ferrofluide VUSPIO fonctionnalisé par de telles espèces F constituent un autre objet spécifique de l'invention.

Les ferrofluides "*VUSPIO fonctionnalisés*" selon l'invention peuvent également être mis en oeuvre pour la préparation de compositions à usage thérapeutique. Ces compositions à usage thérapeutique constituent un autre objet de la présente invention. Dans ce cadre, les espèces F sont (ou comprennent) des principes actifs thérapeutiques, notamment des actifs pharmaceutiques ou bien encore des oligonucléotides. Ces espèces peuvent être alors vectorisées au niveau de cellules ou d'organes spécifiques, la faible taille du ferrofluide permettant de franchir les différentes barrières physiologiques.

De plus, les compositions à usage thérapeutique à base de ferrofluides "*VUSPIO fonctionnalisés*" selon l'invention sont adaptées à la délivrance des principes actifs, avec un suivi de la distribution du principe actif, notamment par IRM. Pour des applications d'administration de principes actifs *in vivo* avec un suivi de la distribution, il s'avère particulièrement avantageux d'utiliser des ferrofluides de type "VUSPIO fonctionnalisés" où les espèces F mises en oeuvre comprennent (i) des actifs thérapeutiques, et (ii) des marqueurs tels que des espèces fluorochromes. Le caractère magnétique de ces particules permet leur localisation globale dans l'organisme, par exemple par IRM, puis une localisation plus précise peut être effectuée, à l'aide des marqueurs de type fluorochrome, qui, par exemple, colorent les tissus où le principe actif est véhiculé, permettant une identification desdits tissus.

De façon générale, les compositions à usage thérapeutique ou à usage diagnostique (agents de contraste pour imagerie médicale) comprenant des dispersions selon l'invention présentant des particules greffées par des espèces d'intérêt diagnostique ou thérapeutique, se présentent le plus souvent sous la forme de compositions administrables par voie orale ou parentérale et, en particulier, sous la forme de compositions injectables. Dans ce cadre, les compositions selon l'invention peuvent comprendre, en plus des particules présentes en dispersion, un ou plusieurs additifs adaptés à l'administration envisagée, et notamment un véhicule adapté au mode d'administration choisi.

Différents avantages et caractéristiques de l'invention ressortiront de façon encore plus nette au vu des exemples illustratifs donnés ci-après.

### EXEMPLE 1:

### Synthèse de dispersions aqueuses de particules nanométriques de maghémite de surface modifiée par couplage d'aminosilanes (ferrofluides aminés)

### 1.1. Synthèse de dispersions aqueuses de particules nanométriques de maghémite (ferrofluides acides)

### 1.1.1. Dispersion (D1)

On a réalisé une dispersion aqueuse (D1) de particules nanométriques de maghémite, en mettant en oeuvre les étapes suivantes :
- Formation de particules de magnétite : on a dissous 31,41 g (soit 0,158 mole) de chlorure ferreux dans 170 ml d'acide chlorhydrique 1,5 M. On a introduit cette solution dans un bêcher de 5 1 contenant 85,4 g (soit 0,316 mole) de chlorure ferrique dissous dans 3,5 l d'eau. On a réalisé une co-précipitation de sels de fer par addition de 200 ml d'une solution d'ammoniaque 2M, à 25° C, sous agitation, ce qui a mené à la formation d'un précipité colloïdal de magnétite. Les particules de magnétite obtenues ont été laissées à décanter sur une plaque magnétique, puis le surnageant a été éliminé.
- Désorption des contre-ions NH₄⁺ et oxydation de surface : le floculat réalisé dans l'étape précédente a été traité pendant une durée de 15 minutes par 200 ml d'acide nitrique de concentration 2M. Ce traitement par l'acide nitrique a été réalisé à la fois pour acidifier la surface des particules en désorbant les contre-ions NH₄⁺ (floculants) et en les remplaçant par des ions nitrates, et pour solubiliser les ions ferreux par oxydation superficielle. Le floculat a ensuite été décanté, et le surnageant a été éliminé à nouveau.
- Oxydation du coeur des particules : on a ensuite introduit 600 ml d'une solution aqueuse de nitrate ferrique de concentration 0,33M préalablement portée à ébullition. On a laissé réagir pendant 30 minutes, puis on a décanté magnétiquement et on a éliminé le surnageant. Dans cette étape, l'apport des ions Fe³⁺ en solution provoque l'oxydation du Fe^{II} des particules, ce qui conduit à la formation d'une phase maghémite γFe₂O₃ au sein des particules.
- Peptisation : On a traité le milieu obtenu par 200 ml d'acide nitrique de concentration 2M. *Lors de cette étape, les protons apportés par l'acide s'adsorbent à la surface des particules d'oxyde, ce par quoi on obtient une charge surfacique permettant une répulsion électrostatique interparticulaire.*
   On a ensuite soumis le milieu à une décantation magnétique, ce par quoi on a obtenu un floculat de particules de maghémite qu'on a lavé trois fois par de l'acétone. On a pris soin, lors de ces lavages, de ne pas laisser sécher le floculat, pour éviter l'agglomération des particules. On a ensuite placé le floculat de maghémite ainsi lavé (et non séché) dans 500 ml d'eau déionisée, ce par quoi on a obtenu une dispersion aqueuse de particules à l'état de sol. L'acétone résiduel a été éliminé par évaporation sous vide à 40° C. Le volume a ensuite été ramené à un litre, par addition d'eau ultrapure à 10 MΩ.

### 1.1.2. Dispersion (D2)

On a réalisé une dispersion aqueuse (D2) de particules de maghémite, en mettant en oeuvre les étapes suivantes :
- Formation de particules de magnétite : on a dissous 31,41 g (soit 0,158 mole) de chlorure ferreux et 127,66 g de nitrate ferrique dans un bécher de 5 litres contenant 2,5 litres d'une solution aqueuse de nitrate de sodium 1M. On a ensuite réalisé une co-précipitation des sels de fer par addition d'une solution d'hydroxyde de sodium de concentration 5M jusqu'à obtention d'un pH de 13,2, ce qui a mené à la formation d'un précipité de magnétite colloïdale qu'on a maintenu sous agitation pendant 15 minutes. Les particules de magnétite obtenues ont été laissées à décanter sur une plaque magnétique, et le surnageant a été éliminé. Le floculat obtenu a ensuite été lavé par deux fois 2 litres d'eau.
- Désorption des contre-ions NH₄⁺ et oxydation de surface : le floculat de l'étape précédente a été traité pendant une durée de 15 minutes par 400 ml d'acide nitrique de concentration 2M.
- Oxydation du coeur des particules : on a porté le milieu obtenu à ébullition, et on y a introduit 600 ml d'une solution aqueuse de nitrate ferrique de concentration égale à 0,33M. On a laissé réagir pendant 30 minutes, ce qui a conduit à la formation d'une phase maghémite γFe₂O₃ au sein des particules, puis on a décanté magnétiquement les particules et on a éliminé le surnageant.
- Peptisation : On a traité le milieu obtenu par 200 ml d'acide nitrique 2M, de façon à obtenir une charge surfacique induisant une répulsion électrostatique interparticulaire.
   On a soumis le milieu à une décantation magnétique, ce par quoi on a obtenu un floculat de particules de maghémite obtenu qu'on a lavé trois fois par de l'acétone, en prenant soin de ne pas laisser sécher le floculat. On a ensuite placé le floculat de maghémite ainsi lavé (et non séché) dans 500 ml d'eau déionisée, ce par quoi on a obtenu une dispersion aqueuse de particules à l'état de sol. L'acétone résiduel a été éliminé par évaporation sous vide à 40° C. Le volume a ensuite été ramené à un litre, par addition d'eau ultrapure à 10 mΩ.

### 1.1.3. Dispersion (D3)

On a réalisé une dispersion (D3) de particules de maghémite en mettant en oeuvre les étapes suivantes :
- Formation de particules de magnétite : on a dissous 31,41 g (soit 0,158 mole) de chlorure ferreux et 127,66 g de nitrate ferrique dans un bécher de 5 litres contenant 2,5 litres d'une solution aqueuse de nitrate de sodium 3M. On a ensuite réalisé une co-précipitation des sels de fer par addition d'une solution d'hydroxyde de sodium de concentration 5M jusqu'à obtention d'un pH de 13,2, ce qui a mené à la formation d'un précipité de magnétite colloïdale qu'on a maintenu sous agitation pendant 15 minutes. Les particules de magnétite obtenues ont été laissées à décanter sur une plaque magnétique, et le surnageant a été éliminé. Le floculat obtenu a ensuite été lavé par deux fois 2 litres d'eau.
- Désorption des contre-ions NH₄⁺ et oxydation de surface : le floculat réalisé dans l'étape précédente a été traité pendant une durée de 15 minutes par 400 ml d'acide nitrique de concentration 2M.
- Oxydation du coeur des particules : on a porté le milieu obtenu à ébullition, et on y a introduit 600 ml d'une solution aqueuse de nitrate ferrique de concentration égale à 0,33M. On a laissé réagir pendant 30 minutes, , ce qui a conduit à la formation d'une phase maghémite γFe₂O₃ au sein des particules, puis on a décanté magnétiquement les particules obtenues et on a éliminé le surnageant.
   Peptisation : On a traité le sol obtenu par 200 ml d'acide nitrique 2M. Lors de cette étape, les protons apportés par l'acide s'adsorbent à la surface des particules d'oxyde, ce par quoi on obtient une charge surfacique permettant une répulsion.
   On a ensuite soumis le milieu obtenu à une décantation magnétique, ce par quoi on a obtenu un floculat de particules de maghémite qu'on a lavé trois fois par de l'acétone, en prenant soin de ne pas laisser sécher le floculat. On a ensuite placé le floculat de maghémite ainsi lavé (et non séché) dans 500 ml d'eau déionisée, ce par quoi on a obtenu une dispersion aqueuse de particules à l'état de sol. L'acétone résiduel est éliminé par évaporation sous vide à 40° C. Le volume a ensuite été ramené à un litre, par addition d'eau ultrapure à 10 MΩ.

Les caractéristiques physico-chimiques des dispersions (D1) à (D3) sont regroupées dans le tableau 1 ci-après.

**Tableau 1: caractéristiques physico-chimiques des dispersions (D1) à (D3)**

| Dispersion | (D1) | (D2) | (D3) |
|---|---|---|---|
| pH | 2,5 | 2,5 | 2,5 |
| Concentration en maghémite | 28 g/l | 28 g/l | 28 g/l |
| Point isoélectrique | 7,3 | 7,3 | 7,3 |
| Diamètre hydrodynamique moyen en nombre MET⁽¹⁾ | 7,8 nm | 4,0 nm | 1,5 nm |
| Diamètre hydrodynamique moyen en nombre PCS⁽²⁾ | 8,5 nm | 2,9 nm | 2 nm |

| | | | |
|---|---|---|---|
| *⁽¹⁾* : *tel qu'estimé par microscopie électronique à transmission* *⁽²⁾ : diamètre hydrodynamique moyen tel que mesuré par spectroscopie de corrélation de photons.* | | | |

### 1.2. Modification de la surface des particules de maghémite au sein des ferrofluides acides synthétisés dans les exemples 1.1.1 à 1.1.3

A partir des ferrofluides acides stabilisés (D1), (D2) et (D3) synthétisés dans les étapes précédentes, on a réalisé différentes dispersions de particules de maghémite de surface modifiée (ferrofluides aminés) notées (D1a), (D1b), (D1c) (D2a) et (D3a). La modification de surface a été réalisée par différents composés aminosilanes en mettant en oeuvre le protocole général suivant :
- Réaction de couplage :
   On a placé un volume de 200 ml du ferrofluide considéré ((D1), (D2) ou (D3), selon le cas), de concentration en Fe³⁺ égale à 0,35M, sous agitation magnétique à une vitesse de 300 tours par minute. On a ajouté sous agitation 100 ml de méthanol technique à ce ferrofluide. On a ensuite ajouté au mélange, maintenu sous agitation, une solution, dans 100 ml de méthanol, d'une quantité de composé aminosilane correspondant à 148,3 micromoles de silane par m² de surface développée par les particules du ferrofluide considéré, à savoir 0,1 mole pour le ferrofluide (D1), 0,2 mole pour (D2) et 0,5 mole pour (D3). On a laissé la réaction se poursuivre pendant 12 heures.
- Traitement thermique (déshydratation)
   Suite à l'étape précédente, on a ajouté 200 ml de glycérol et on a homogénéisé le milieu pendant quelques minutes sous une agitation de 500 tours par minute. On a ensuite extrait le méthanol et l'eau à l'aide d'un évaporateur rotatif (extraction sous vide primaire pendant une heure, à 40° C pour le méthanol, puis à 80° C pour l'eau).
   Le milieu obtenu a ensuite été soumis à un traitement thermique à 100°C sous vide secondaire (pompe à palettes) pendant 2 heures. On a ensuite laissé le milieu refroidir jusqu'à la température ambiante.
- Extraction, lavage
   Le mélange obtenu à l'issue du traitement thermique (particules modifiées de maghémite dans un milieu essentiellement constitué de glycérol, contenant en outre du silane n'ayant pas réagi) a été introduit dans un bêcher dans lequel on a versé successivement 100 ml d'éthanol, puis 200 ml d'acétone sous agitation lente (100 tours par minute) de façon à réaliser une floculation des particules de maghémite modifiées, ainsi qu'une précipitation de l'excès de silane oligomérisé.
   On a réalisé une décantation des particules sur une plaque magnétique, et on a éliminé le surnageant comprenant le silane oligomérisé. Le floculat de particules obtenu a été lavé par trois fois 400 ml d'un mélange (acétone/eau ultra pure) (70:30 v/v), de façon à éliminer les oligomères de silane et le glycérol résiduels. Là encore, les lavages ont été effectués de façon à ne pas laisser sécher le floculat.

### Peptisation

Après le dernier lavage de l'étape précédente, on a ajouté au floculat obtenu 400 ml d'eau ultra pure. Le pH a alors été mesuré égal à 10,4, attestant de la présence de fonctions amine à la surface de la maghémite, et de la saturation de cette surface par les fonctions amines.

On a alors ajouté au milieu, goutte à goutte, et sous agitation élevée (de l'ordre de 700 tours par minute), de l'acide nitrique 1M, de façon à faire progressivement diminuer le pH, pas à pas, d'environ une unité de pH à chaque étape. Cette acidification permet en particulier de préserver l'adhésion du film de polysiloxane aminé qui a été formé dans les étapes précédentes sur la surface des particules.

Lorsqu'on a atteint un pH de 6, les particules de maghémite ont commencé à se disperser. On a ajusté le pH du milieu à 3 et on a laissé le milieu sous agitation une journée. Le pH a ensuite été à nouveau réajusté à 3.

Dans ces conditions, on a réalisé les 5 dispersions suivantes (ferrofluides aminés) :

| Ferrofluide acide de départ | (D1) | (D1) | (D1) | (D2) | (D3) |
|---|---|---|---|---|---|
| Composé aminosilane utilisé pour la modification | APS ⁽³⁾ | EDPS ⁽⁴⁾ | DTPS ⁽⁵⁾ | APS | APS |
| Ferrofluide aminé obtenu | (D1a) | (D1b) | (D1c) | (D2a) | (D3a) |

| | | | | | |
|---|---|---|---|---|---|
| *⁽³⁾ : APS :* γ*-aminopropyltriméthoxysilane* *⁽⁴⁾ : EDPS : N-*β*-(aminoéthyl)-*γ*-aminopropyltriméthoxysilane* *⁽⁵⁾ : DTPS : N-*β*-(aminoéthyl)-N'-*β*-(aminoéthyl)-γ-aminopropyltriméthoxysilane.* | | | | | |

### 1.3. Propriétés des dispersions de particules de maghémite modifiées (ferrofluides aminés) obtenus

Les caractéristiques des 5 ferrofluides aminés obtenus sont regroupées dans le tableau 2 ci-dessous.

**Tableau 2 : Propriétés des ferrofluides aminés obtenus**

| Ferrofluide aminé | (D1a) | (D1b) | (D1c) | (D2a) | (D3a) |
|---|---|---|---|---|---|
| pH | 3 | 3 | 3 | 3 | 3 |
| Concentration en Fe | 0,35M | 0,35M | 0,35M | 0,35M | 0,35M |
| Point isoélectrique | 10,4 ⁽⁶⁾ | 10,2 | 10,1 | 10,4 | 10,4 |
| Diamètre hydrodynamique moyen en nombre PCS | 15,2 nm | 7,7 nm | 15,5 nm | 6,0 nm | 5,7 nm |

| | | | | | |
|---|---|---|---|---|---|
| *⁽⁶⁾ : A titre de comparaison,* on *a réalisé une dispersion en modifiant la dispersion D1 par de l'APS dans les conditions du protocole général décrit ci-dessus, mais sans mettre en oeuvre la deuxième étape de traitement thermique. Le point isoélectrique de la composition a alors été mesuré égal à 8,3.* | | | | | |

### EXEMPLE 2 : Stabilité vis-à-vis de la floculation en présence d'ions chlorure

Pour illustrer la stabilité accrue des ferrofluides aminés de l'invention par rapport aux ferrofluides acides en ce qui concerne la floculation, on a réalisé un test comparatif consistant à introduire des ions chlorure à des concentrations croissantes au sein des deux types de ferrofluides.

Le tableau 3 ci-dessous montre la concentration limite de floculation observée à pH = 3, pour les dispersions (D1), (D1a), (D1b) et (D1c) de l'exemple 1. La concentration limite de floculation est la concentration minimale en ions chlorure dans la dispersion, au pH considéré, à partir de laquelle on observe un début de floculation, à savoir une augmentation de la turbidité, due à la formation et à l'accroissement de la taille des floculats dans le milieu. La concentration limite correspond à la concentration à laquelle on observe une augmentation de la densité optique, mesurée à une longueur d'onde de 800 nm.

**Tableau 3 : Concentration limite de floculation lors de l'ajout d'ions chlorure**

| Dispersion | (D1) | (D1ₐ) | (D1_{b}) | (D1_{c}) |
|---|---|---|---|---|
| Concentration limite de floculation (Cl-)^{(*)} | 0,05 mol/l | 0,14 mol/l | 0,17 mol/l | 0,21 mol/l |

On voit, au vu des données ci-dessus, que la modification de surface permet de multiplier au moins par deux la concentration limite de floculation en présence d'ions chlorure.

### EXEMPLE 3 : Greffage de molécules de dextrane (Synthèse de ferrofluides aminés modifiés de type "VUSPIO")

On a réalisé le greffage de molécules de dextrane sur les particules des ferrofluides aminés de l'exemple 1.2 . Quatre classes différentes de dextrane ont été testés (Dextrane T5 : *M̅_̅{̅w̅}̅ =5* 000 ; Dextrane T15 : *M̅_{w}* =15 000 ; Dextrane T40 : *M̅_̅{̅w̅}̅* =40 000 ; et Dextrane T70 : *M̅_{w}* =70 000).

Le greffage a été réalisé selon le protocole suivant :
- Activation du dextrane :
   On a réalisé une solution de 10 g de dextrane dans 200 ml d'eau ultrapure. On a ajouté à ce milieu 10 ml d'une solution aqueuse de NaIO₄ à 2,06 mol.l⁻¹ et on a laissé le mélange 12 h sous agitation. A l'issue de cette étape d'oxydation, on a éliminé les sels périodiques obtenus dans le milieu. Pour ce faire, la solution obtenue, jaune pâle, a été versée dans un tube à dialyse en cellulose (seuil de coupure 12 400 g/mol⁻¹, Aldrich). La dialyse a été realisée dans un bêcher de 5 1 contenant de l'eau ultrapure. L'eau a été renouvelée 5 fois toutes les 2 heures. La solution de dextrane activé obtenue a été stockée à 4°C. La présence d'aldéhydes a été confirmée par le test de Fehling.
- Greffage du dextrane activé sur les particules d'un ferrofluide aminé
   Un volume de 200 ml de la solution de dextrane activé à 10 g/l précédemment obtenue, a été versé dans 20 ml d'un ferrofluide tel qu'obtenu dans l'exemple 1.2. Le mélange réalisé a été laissé sous agitation pendant 24 h, puis on a ajouté 10 ml d'une solution de borohydrure de sodium à 0,206 mol.l⁻¹. Le milieu obtenu a été laissé sous agitation pendant 4 h à pH = 9.
   Dans tous les cas, on a obtenu un sol stable.
- Purification du sol obtenu par ultrafiltration tangentielle :
   On a utilisé un système d'ultrafiltration constitué d'une pompe péristaltique (Millipore N80EL005), de tuyauterie en silicone et d'une cartouche (Prep/Scale™-TFF) renfermant une membrane en poly(éther sulfone) de seuil de coupure égal à 100 kD.
   Le sol a été placé dans le réservoir de rétentat. Après passage du sol dans la membrane, on a versé 11 d'eau dans le réservoir afin de procéder au lavage. Le sol a été lavé et neutralisé contre 3 1 d'eau ultrapure. Le sol purifié qui a été obtenu est plus dilué que le sol initial car le volume mort du système est de 100 ml environ. Une partie du produit reste colmaté à l'intérieur de la membrane. Celui-ci est cependant évacué après plusieurs heures de lavage. Par évaporation, on a concentré la dispersion de particules à une concentration en Fe³⁺ de 0,08M.

Le tableau ci-dessous regroupe les résultats obtenus par greffage de différents dextranes sur les ferrofluides aminés (D1a), (D2a) et (D3a) de l'exemple 1.2. Les différents ferrofluides aminés modifiés obtenus sont indicés (G1) à (G9).

**Tableau 4 : ferrofluides aminés modifiés par greffage de dextranes.**

| ferrofluide modifié réalisé | ferrofluide aminé de départ | dextrane utilisé pour le greffage | Diamètre hydrodynamique moyen en nombre (PCS) des particules du ferrofluide modifié réalisé |
|---|---|---|---|
| (G1) | (D1a) | T70 | 59,6 nm |
| (G2) | (D1b) | T70 | 130 nm |
| (G3) | (D1c) | T70 | 130 nm |
| (G4) | (D1a) | T40 | 53,7 nm |
| (G5) | (D1a) | T15 | 132,8 nm |
| (G6) | (D2a) | T40 | 70,8 nm |
| (G7) | (D2a) | T15 | 46,7 nm |
| (G8) | (D2a) | T5 | 44,9 nm |
| (G9) | (D3a) | T40 | 56,5 nm |
| (G10) | (D3a) | T15 | 34,0 nm |
| (G11) | (D3a) | T5 | 32,2 nm |

### EXEMPLE 4 : Synthèse de ferrofluides « VUSPIO » fonctionnalisés

### 4.1. Greffage d'un poly(oxyde d'éthylène) aminotéléchélique (diaminé) sur les particules des ferrofluides modifiés de l'exemple 3.

On a réalisé le greffage de molécules de poly(oxyde d'éthylène) diaminé de dextrane sur les particules des ferrofluides aminés modifiés (G1) à (G3) de l'exemple 3.

Le greffage a été réalisé selon le protocole suivant :
A un volume de 200 ml du ferrofluide considéré ((G1), (G2) ou (G3) selon le cas), on a ajouté une solution de 20,5 g de POE diaminé (M_{w} = 2000 g/mol) dans 100 ml d'eau. On a ensuite ajouté au milieu 100 ml d'une solution aqueuse de borohydrure de sodium de concentration égale à 0,206 mol/l. On a laissé réagir pendant 4 heures à pH 9 et sous agitation, et on a ensuite éliminé l'excès de POE diaminé, sous ultrafiltration tangentielle contre 5 1 d'eau ultrapure. La dispersion de borohydrure et de borate obtenue a été concentrée par évaporation de l'eau jusqu'à obtention d'une concentration en Fe³⁺ de 0,08M.

Le tableau V ci-dessous donne les résultats obtenus dans les trois cas.

**Tableau V : Ferrofluides modifiés par greffage de POE diaminé.**

| Ferrofluides de départ | Diamètre hydrodynamique moyen en nombre (PCS) des particules du ferrofluide réalisé |
|---|---|
| (G1) | 59,6 nm |
| (G2) | 53,7 nm |
| (G3) | 132,8 nm |

### 4.2. Greffage d'un poly(oxyde d'éthylène) monoaminé sur les particules de ferrofluides modifiés de l'exemple 3

On a réalisé un greffage similaire à celui de l'exemple 4 en utilisant les ferrofluides aminés (G4), (G6) et (G9) de l'exemple 3, en mettant en oeuvre pour ce faire le protocole opératoire de l'exemple 4, hormis le fait qu'on a utilisé un POE monoaminé (Mw = 2000 g/mol). Par ailleurs, la quantité de POE a été divisée par deux par rapport à l'exemple 4. Ainsi, la solution de POE ajoutée contient 10,25 g de POE monoaminé dans 100 ml d'eau.

Le tableau VI ci-dessous regroupe les résultats obtenus par le greffage des ferrofluides aminés D1a, D2a et D3a de l'exemple 1.2.

**Tableau VI : Ferrofluides modifiés par greffage de POE monoaminé**

| Ferrofluide aminé de départ | Diamètre hydrodynamique moyen en nombre (PCF) de particules de ferrofluide modifié réalisées |
|---|---|
| (G4) | 74,5 nm |
| (G6) | 62,1 nm |
| (G9) | 65,6 nm |

### 4.3. Greffage des particules des ferrofluides modifiés de l'exemple 3 par des espèces fluorescentes

On a laissé réagir pendant 24 heures, à l'abri de la lumière, 100 ml des ferrofluides modifiés de l'exemple 3, de concentration en Fe³⁺ égale à 0,08M, tamponnés par un tampon phosphate (pH 7,4 ; 0,01M) avec 10⁻³ mol de Rhodamine B (Rh B) ou de Lucifer Yellow (LY) sous la forme de son sel dilithié. On a ensuite effectué une réduction au borohydrure, dans les conditions de l'exemple 3. Ces espèces aminées réagissent directement avec les fonctions aldéhyde des dextranes oxydés. Suite à cette réaction, on a éliminé l'excès d'espèces fluorescentes (fluorochromes) par extraction liquide-liquide (eau/chloroforme) jusqu'à ce que la phase organique devienne incolore. Les traces de chloroforme sont ensuite éliminées à l'évaporateur rotatif.

### 4.4 Greffage des particules des ferrofluides modifiés de l'exemple 4.1 par des espèces fluorescentes

On a effectué la réaction de couplage sur 100 ml de ferrofluide selon l'exemple 4.1, de concentration en Fe³⁺ égale à 0,08M, tamponné par un tampon carbonate/bicarbonate de sodium (pH 9, 0,01 m) par 10⁻³ moles de TRITC (dérivé isothiocyanate de la tétraméthylrhodamine) ou de (5-FAM, SE) (l'ester succinimidique de la 5-carboxyfluorescéine). Le (5-FAM, SE) a été préalablement dissous dans 3 ml de DMF. L'élimination de l'excès des espèces fluorescentes a été effectuée par extraction liquide-liquide (eau/chloroforme), jusqu'à ce que la phase organique devienne incolore. Les traces de chloroforme ont été éliminées à l'évaporateur rotatif.

### 4.5: Greffage des particules des ferrofluides modifiés de l'exemple 6 par des espèces fluorescentes

Le mode opératoire utilisé est identique à celui de l'exemple 4.4 sauf que le POE diaminé est remplacé par un mélange de POE monoamine et de POE diaminé dans les proportions 2:1.

### 4.6: Greffage de la doxorubicine sur les particules des ferrofluides des exemples 4.1 et 4.2

2,22.10⁻⁴ mole de doxorubicine (nombre de mole équivalent à 1/30^{ème} de moles de résidus glucosidique) a été ajouté dans la dispersion de particules nanométriques préparées dans l'exemple 3 avant la réduction au borohydrure. Le mode opératoire décrit dans l'exemple 3 est ensuite suivi. Les particules traitées à la doxorubicine sont alors soumis au même traitement que celles issues de l'exemple 4.1 ou 4.2.

### 4.7: Greffage de l'acide folique sur les particules des ferrofluides de l'exemple 3.

### 4.7.1 Estérification de l'acide folique avec le N-Hydroxysuccinimide (NHS):

5 g d'acide folique ont été dissous dans 100 ml de DMSO. On a ajouté à ce mélange 2,5 ml de triéthylamine et 2,6 g de NHS et 4,7 g carbodiimide. On a laissé réagir une nuit à température ambiante. Le co-produit de la réaction, le dicyclohexylurée est extrait par filtration. La solution d'acide folique couplée au NHS est concentrée à l'évaporateur sous pression réduite. Le produit est alors précipité dans le diéthyléther.

### 4.7.2 Conjugaison de POE diaminé avec l'acide folique couplé au NHS:

15 g de POE diaminé (M_{w}=2000 g/mol) ont été dissous dans 100ml d'une solution tampon carbonate/bicarbonate à pH 10,5. 4,5 g du produit (acide folique-NHS) obtenu à l'issue de l'étape 4.7.1 a été dissous dans un minimum de DMSO (10ml). Cette solution a alors été versée goutte à goutte à la solution contenant le POE diaminé, et on a laissé réagir pendant 12 h. Le produit a alors été dialyse contre de l'eau ultrapure dans un tube à dialyse de 1kD de seuil de coupure, pendant 12h, en changeant l'eau régulièrement.

### 4.7.2 Greffage du conjugué de l'étape 4.7.2 sur les particules de l'exemple 3:

Le mode opératoire est identique à celui de l'exemple 4.5 en remplaçant le POE monoaminé par le conjugué POE-acide folique de l'étape 4.7.2.

## Revendications

1. Dispersion aqueuse comprenant des particules (p) à base d'un oxyde de fer magnétique, de dimensions inférieures ou égale à 20 nm, dont la surface est modifiée par le greffage de groupements aminés R liés de façon covalente à la surface desdites particules, dans laquelle le point isoélectrique des particules de surface ainsi modifiée est supérieur ou égal à 10.

2. Dispersion aqueuse selon la revendication 1, **caractérisée en ce qu'**elle a un pH inférieur ou égal à 8 et **en ce qu'**elle se présente sous la forme d'une dispersion de particules ayant un diamètre hydrodynamique moyen inférieur ou égal à 20 nm.

3. Dispersion selon la revendication 2, **caractérisée en ce que** le diamètre hydrodynamique moyen des particules (p) est compris entre 3 et 15 nm.

4. Dispersion selon l'une quelconque des revendications 1 à 3, **caractérisée en ce que** les particules (p) sont essentiellement constituées de maghémite (Fe₂O₃-γ), de préférence monocristalline.

5. Dispersion selon l'une quelconque des revendications 1 à 4, **caractérisée en ce que** les groupements aminés R sont des groupements de formule -(A)-NH₂, dans laquelle le groupement -(A)- désigne une chaîne hydrocarbonée comportant de 1 à 12 atomes de carbone, éventuellement interrompue par un ou plusieurs groupements -NH-.

6. Dispersion selon la revendication 5, **caractérisée en ce que** les groupements aminés R sont choisis parmi :
(i) les groupements de formule -(CH₂)n₁-NH₂, où n₁ = 1, 2, 3, 4, 5, 6, 7 ou 8;
(ii) les groupements de formule -(CH₂)n₂-NH-(CH2)n₂, -NH₂, où n2 et n₂' sont identiques ou différents et désignent chacun 1, 2, 3, 4, 5 ou 6, étant entendu que (n₂ + n_{2'}) reste compris entre 2 et 9 ;
(üi) les groupements de formule -(CH₂)n₃-NH-(CH₂)n_{3'} -NH-(CH₂)n_{3'}-NH₂, où n₃, n_{3'} et n_{3"} sont identiques ou différents et désignent chacun 1, 2, 3, ou 4, étant entendu que (n₃ + n_{3'} + n_{3"}) reste compris entre 3 et 12

7. Dispersion selon l'une quelconque des revendications 1 à 6, **caractérisée en ce que** les groupements aminés R sont liés à la surface des particules (p) par l'intermédiaire d'une liaison :

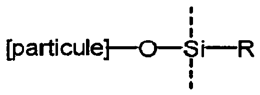

8. Procédé de préparation d'une dispersion selon l'une quelconque des revendications 1 à 7, comprenant les étapes consistant à :
(A) fournir une dispersion aqueuse acide de particules (po) à base d'un oxyde de fer magnétique de dimensions inférieures à 20 nm, ladite dispersion présentant, en milieu acide, une stabilité colloïdale au moins dans une gamme de pH, cette stabilité étant telle que, dans ladite gamme de pH, on observe, sans avoir à maintenir une agitation, une dispersion de particules ayant un diamètre hydrodynamique moyen inférieur à 20 nm où moins de 5% en nombre des espèces solides observées en suspension sont des agglomérats de plusieurs particules ;
(B) mettre en contact la dispersion colloïdale acide de l'étape (A) avec des silanes de formule R-SiX₁X₂X₃, dans laquelle :
- R désigne un groupement aminé tel que défini dans la revendication 1 ou dans l'une des revendications 5 ou 6 ;
- X₁, X₂, et X₃ sont des groupements identiques ou différents, désignant chacun un groupe hydrolysable en milieu acide,
cette mise en contact étant effectuée en maintenant le milieu dans la gamme de pH où la stabilité colloïdale de la dispersion est assurée ;
(C) ajouter au milieu réactionnel un agent mouillant hydrosoluble de température d'ébullition supérieure à celle de l'eau, puis chauffer le milieu réactionnel à une température suffisante pour éliminer l'eau, mais sans éliminer l'agent mouillant ; et
(D) récupérer les particules obtenues à l'issue de l'étape (C) et les disperser en milieu aqueux.

9. Procédé selon la revendication 8, **caractérisé en ce que** la dispersion aqueuse acide colloïdale de l'étape (A), est telle que, dans la gamme de pH où la stabilité colloïdale est assurée, le diamètre hydrodynamique moyen des particules observées en suspension est compris entre 3 et 15 nm.

10. Procédé selon la revendication 8 ou selon la revendication 9, **caractérisé en ce que** la dispersion aqueuse acide colloïdale de l'étape (A), est telle que, dans la gamme de pH où la stabilité colloïdale est assurée, moins de 5% en nombre des espèces solides observées en suspension sont des agglomérats de plusieurs particules.

11. Procédé selon l'une quelconque des revendications 8 à 10, **caractérisé en ce que** les silanes mis en oeuvre dans L'étape (B) sont des trialcoxysilanes aminés de formule R-Si(OR')(OR")(OR"') dans laquelle :
- R est un groupe aminé tel que défini dans la revendication 8; et
- R', R" et R"', identiques ou différents, désignent chacun un groupement alkyle comportant de 1 à 5 atomes de carbone.

12. Procédé selon la revendication 11, **caractérisé en ce que** les silanes de l'étape (B) sont choisis parmi :
- le γ-aminopropyltriméthoxysilane, de formule :
(CH₃O)₃-Si-(CH₂)₃-NH₂ ;
- le N-β-aminoéthyl-γ-aminopropyltriméthoxysilane, de formule :
(CH₃O)₃-Si-(CH₂)₃-NH-(CH₂)₂-NH₂ ;
- le N'-β-aminoéthyl-N-β-aminoéthyl-γ-aminopropyltriméthoxysilane, de formule :
(CH₃O)₃-Si-(CH₂)₃-NH-(CH₂)₂-NH-(CH₂)₂-NH₂.

13. Procédé selon l'une quelconque des revendications 8 à 12, **caractérisé en ce que** les silanes de l'étape (B) sont introduits en solution dans un solvant organique, **en ce que** l'agent mouillant de l'étape (C) est soluble dans le solvant organique solubilisant les silanes introduits dans l'étape (B) et possède une température d'ébullition supérieure à celle dudit solvant organique, et **en ce que** l'étape (C) comprend une étape de chauffage à une température suffisante pour éliminer ledit solvant organique sans éliminer l'agent mouillant.

14. Procédé selon l'une quelconque des revendications 8 à 13, **caractérisé en ce que**, dans l'étape (C), l'agent mouillant est le glycérol.

15. Procédé selon l'une quelconque des revendications 8 à 14, **caractérisé en ce que** le chauffage de l'étape (C) est conduit sous vide.

16. Procédé selon la revendication 15, **caractérisé en ce que** la déshydratation de l'étape (C) est conduite à une température inférieure ou égale à 130°C.

17. Procédé selon l'une quelconque des revendications 8 à 16, **caractérisé en ce que** l'étape (D) comprend un lavage des particules obtenues à l'issu de l'étape (C), effectué sans laisser sécher les particules, suivi d'une dispersion du floculat de particules non séché obtenu en milieu aqueux.

18. Procédé selon l'une quelconque des revendications 8 à 17, **caractérisé en ce que** la dispersion des particules effectuée lors de l'étape (D) est réalisée en plaçant les particules récupérées à l'issue de l'étape (C) dans de l'eau et en diminuant progressivement le pH du milieu, par addition lente d'un acide.

19. Utilisation d'une une dispersion selon l'une quelconque des revendications 1 à 7, pour la préparation d'une composition administrable par voie orale ou parentérale chez l'homme ou chez l'animal, et notamment pour la préparation de compositions injectables d'agents de contraste pour l'imagerie par résonance magnétique.

20. Composition pour une administration chez l'homme ou l'animal comprenant une dispersion selon l'une quelconque des revendications 1 à 7.

21. Utilisation d'une dispersion selon l'une quelconque des revendications 1 à 7, à titre de charge magnétique dans une composition ou un matériau magnétique.

22. Procédé selon la revendications 8, comprenant, suite aux étapes (A), (B), (C) et (D), une étape (G1) consistant à faire réagir ladite dispersion selon l'une des revendications 1 à 7 avec des espèces chimiques E susceptibles de former une liaison avec les groupements aminés R présents à la surface des particules (p), à un pH inférieur à 8, ce par quoi on obtient une dispersion selon la revendication 1 modifiée en surface par lesdites espèces E.

23. Procédé selon la revendication 22, **caractérisé en ce que** les espèces chimiques E présentent des groupements aldéhyde, **en ce que** les groupements aminés R présentent des groupements -NH₂, et en ce, l'étape (G1) consiste à faire réagir la dispersion selon l'une des revendications 1 à 9 avec des espèces chimiques E porteuses de groupements -CHO en présence d'un agent réducteur.

24. Procédé selon la revendication 23, **caractérisé en ce que** les espèces E sont des molécules de polysaccharides dont une partie des groupements -OH a été oxydée en groupement -CHO.

25. Procédé selon la revendication 24, **caractérisé en ce que** les espèces E sont des molécules de dextrane dont une partie des groupements -OH a été oxydée en groupements -CHO.

26. Dispersion aqueuse selon l'une des revendications 1 à 7, dans laquelle des espèces chimiques E, qui sont des polysaccharides, par exemple des molécules de dextrane, dont une partie des groupements -OH a été oxydée en groupements-CHO, sont immobilisées à la surface modifiée des particules, ladite dispersion susceptible d'être obtenue selon le procédé de l'une quelconque des revendications 22 à 25.

27. Dispersion selon la revendication 26, **caractérisée en ce qu'**au moins 90% en nombre des éléments solides qu'elle contient sont des particules individualisées comprenant un noyau central unique, à base d'un oxyde de fer magnétique, ayant des dimensions inférieures à 20 nm.

28. Dispersion aqueuse selon la revendication 27, qui est une dispersion aqueuse de particules à base d'un oxyde de fer magnétique à la surface desquelles sont immobilisées par liaison covalente, des molécules de polysaccharide liées à la surface, par l'intermédiaire de liaisons covalentes de formule -NH-CH₂-, ladite suspension étant susceptible d'être obtenue selon le procédé de la revendication 24.

29. Dispersion aqueuse selon la revendication 27, qui est une dispersion aqueuse de particules à base d'un oxyde de fer magnétique à la surface desquelles sont immobilisées par liaison covalente des molécules de dextrane, par l'intermédiaire de liaisons covalente -NH-CH₂-, cette dispersion étant susceptible d'être obtenue selon le procédé de la revendication 25.

30. Dispersion selon la revendication 28 ou selon la revendication 29, dans laquelle le diamètre hydrodynamique moyen des particules de surface modifiée par les molécules de polysaccharide est inférieur à 50 nm.

31. Dispersion selon l'une quelconque des revendications 28 à 30, dans laquelle au moins 90% des éléments solides en suspension sont des particules individualisées comprenant un noyau central unique, à base d'un oxyde de fer, ayant des dimensions inférieures à 20 nm, ce noyau étant entouré par une couche comprenant les molécules de polysaccharides liées de façon covalente.

32. Dispersion selon l'une quelconque des revendications 28 à 31, dans laquelle les particules à base d'oxyde de fer magnétique sont essentiellement constituées de maghémite (Fc₂O₃-γ).

33. Dispersion selon l'une quelconque des revendications 28 à 32, dans laquelle une partie des groupements -OH des molécules de polysaccharide immobilisées à la surface des particules sont oxydés sous la forme de groupements -CHO.

34. Utilisation d'une dispersion selon l'une quelconque des revendications 28 à 33, pour la préparation d'une composition d'agents de contraste pour l'imagerie par résonance magnétique.

35. Composition d'agents de contraste pour l'imagerie par résonance magnétique comprenant une dispersion selon l'une quelconque des revendications 28 à 33.

36. Procédé selon la revendication 24, comprenant en outre une étape (C2) consistant à faire réagir une dispersion selon l'une des revendications 28 à 33 avec des espèces chimiques F susceptibles de former une liaison avec les molécules de polysaccharide.

37. Procédé selon la revendication 36 où l'étape (G2) consiste à faire réagir une dispersion selon la revendication 33 avec des espèces chimiques F ayant un groupement -NH₂, et à traiter le milieu obtenu par un agent réducteur.

38. Dispersion aqueuse de particules selon la revendication 26, **caractérisée en ce qu'**elle est à base d'un oxyde de fer magnétique à la surface desquelles sont immobilisées par liaison covalente des molécules de dextrane, par l'intermédiaire de liaisons covalente de formule -NH-CH2-, ces molécules de dextrane étant elle-mêmes liés à des espèces chimique F, cette dispersion étant susceptible d'être obtenue selon le procédé de la revendications 36 ou 37.

39. Composition d'agent de contraste pour imagerie médicale ayant une affinité pour des cellules, des tissus ou des organes donnés, **caractérisée en ce qu'**elle comprend une dispersion selon la revendication 38 dans laquelle les espèces F sont des espèces présentant une affinité vis-à-vis desdites cellules, desdits tissus ou desdits organes.

40. Composition à usage thérapeutique, **caractérisée en ce qu'**elle comprend une dispersion selon la revendication 38 dans laquelle les espèces F sont des principes actifs thérapeutiques.

41. Composition selon la revendication 39 ou selon la revendication 40, **caractérisée en ce qu'**elle se présente sous la forme d'une composition injectable.

42. Utilisation d'une dispersion selon l'une quelconque des revendications 1 à 7, pour la préparation d'une suspension de particules magnétiques essentiellement individualisées à la surface desquelles sont immobilisées des espèces chimiques, où l'immobilisation des espèces chimiques est réalisée en établissant une liaison entre lesdites espèces chimiques et les groupements aminés R présents à la surface des particules (p).

## Claims

1. An aqueous dispersion comprising particles (p) based on a magnetic iron oxide having dimensions of less than or equal to 20 nm, the surface of said particles being modified by the grafting of aminated groups R covalently bonded to the surface of the particles, wherein the isoelectric point of the particles with the surface so modified being greater than or equal to 10.

2. The aqueous dispersion as claimed in claim 1, **characterized in that** it has a pH of less than or equal to 8 and **in that** it is provided in the forum of a dispersion of particles having an average hydrodynamic diameter of less than or equal to 20 nm.

3. The dispersion as claimed in claim 2, **characterized in that** the average hydrodynamic diameter of the particles (p) is between 3 and 15 nm.

4. The dispersion as claimed in any one of claims 1 to 3, **characterized in that** the particles (p) are composed essentially of maghemite (γ-Fe₂O₃), preferably monocrystalline maghemite.

5. The dispersion as claimed in any one of claims 1 to 4, **characterized in that** the aminated groups R are groups of formula -(A)-NH₂ in which the -(A)- group denotes a hydrocarbon chain comprising from 1 to 12 carbon atoms which is optionally interrupted by one or more -NH- groups.

6. The dispersion as claimed in claim 5, **characterized in that** the aminated groups R are selected from:
(i) the groups of formula (CH₂)n₁-NH₂- where n₁ = 1, 2, 3, 4, 5, 6, 7 or 8;
(ii) the groups of formula -(CH₂)ₙ₂-NH-(CH₂)_{n2'}-NH₂, where n₂ and n₂, are identical or different and each denote 1, 2, 3, 4, 5 or 6, it being understood that (n₂ + n_{2'}) remains between 2 and 9;
(iii) the groups of formula -(CH₂)ₙ₃-NH-(CH₂)ₙ₃, -NH-(CH₂)_{n3"}-NH₂, where n₃, n₃, and n_{3"} are identical or different and each denote 1, 2, 3 or 4, it being understood that (n₃ + n_{3'} + n_{3"}) remains between 3 and 12.

7. The dispersion as claimed in any one of claims 1 to 6, **characterized in that** the aminated groups R are bonded to the surface of the particles (p) via a bond:

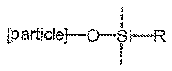

8. A process for the preparation of a dispersion as claimed in any one of claims 1 to 7, comprising the stages consisting in:
(A) providing an acidic aqueous dispersion of particles (p₀) based on a magnetic iron oxide with dimensions of less than 20 nm, said dispersion exhibiting, in an acidic medium, a colloidal stability at least within a pH range, this stability being such that, within said pH range, a dispersion of separate particles having an average hydrodynamic diameter of less than 20 nm is observed wherein less than 5% by number of the solid entities which are observed in suspension are agglomerates of several particles, without having to keep stirred;
(B) bringing the acidic colloidal dispersion of stage (A) into contact with silanes of formula R-SiX₁X₂X₃, in which:
- R denotes an aminated group as defined in claim 1 or in either of claims 5 and 6;
- X₁, X₂ and X₃ are identical or different groups each denoting a group which can be hydrolyzed in an acidic medium,
this contracting operation being carried out while maintaining the medium within the pH range where the colloidal stability of the dispersion is ensured;
(C) adding, to the reaction medium, a water-soluble wetting agent with a boiling point greater than that of water and then heating the reaction medium to a temperature sufficient to remove the water but without removing the wetting agent; and
(D) recovering the particles obtained on conclusion of stage (C) and dispersing them in an aqueous medium.

9. The process as claimed in claim 8, **characterized in that** the acidic aqueous colloidal dispersion of state (A) is such that, in the pH range where the colloidal stability is ensured, the average hydrodynamic diameter of the particles which are observed in suspension is between 3 and 15 nm.

10. The process as claimed in claim 8 or as claimed in claim 9, **characterized in that** the acidic aqueous colloidal dispersion of stage (A) is such that, in the pH range where the colloidal stability is ensured, less than 5% by number of the solid entities which are observed in suspension are agglomerates of several particles.

11. The process as claimed in any one of claims 8 to 10, **characterized in that** the silanes employed in stage (B) are laminated trialkoxysilanes of formula R-Si(OR') (OR") (OR"') in which:
- R is an aminated group as defined in claim 8; and
- R', R" and R"', which are identical or different, each denote an alkyl group comprising from 1 to 5 carbon atoms.

12. The process as claimed in claim 11, **characterized in that** the silanes of stage (B) are selected from:
- γ-aminopropyltrimethoxysilane, of formula:
(CH₃O)₃-Si-(CH₂)₃-NH₂;
- N-(β-aminoethyl)-γ-aminopropyltrimethoxysilane, of formula:
(CH₃O)₃-Si- (CH₂)₃-NH-(CH₂)₂-NH₂;
- N'-(β-aminoethyl)-N-(β-aminoethyl)-γ-aminopropyltrimethoxysilane, of formule:
(CH₃O)₃-Si-(CH₂)₃-NH-(CH₂)₂-NH-(CH₂)₂-NH₂.

13. The process as claimed in any one of claims 8 to 12, **characterized in that** the silanes of stage (B) are introduced in solution in an organic solvent, **in that** the wetting agent of stage (C) is soluble in the organic solvent-which dissolves the silanes introduced in stage (B) and has a boiling point greater than that of said organic solvent and **in that** stage (C) comprises a stage of heating at a temperature sufficient to remove said organic solvent without removing the wetting agent.

14. The process as claimed in any one of claims 8 to 13, **characterized in that**, in stage (C), the wetting agent is glycerol.

15. The process of claim, wherein the heating of stage (C) is carried out under vacuum.

16. The process of claim 15, wherein the dehydration of stage (C) is carried out at a temperature of less than or equal to 130°C.

17. The process of claim 8, wherein stage (D) comprises a washing of the particles obtained on conclusion of stage (C), carried out without allowing the particles to dry, followed by dispersion, in an aqueous medium, of the undried flocculate of particles which is obtained.

18. The process of claim 8, wherein the dispersion of the particles which is produced during stage (D) is produced by placing the particles recovered on conclusion of stage (C) in water and by gradually reducing the pH of the medium by slow addition of an acid.

19. Use of the dispersion as claimed in any one of claims 1 to 7, for the preparation of compositions which can be administered orally or parenterally to animals or man, and in particular, for the reparation of injectable compositions of contrast agents for magnetic resonance imaging (MRI).

20. A composition for administration to man or animal, comprising a dispersion as claimed in any one of claims 1 to 7.

21. Use of a dispersion according to any ore of claims 1 to 7, as a magnetic filler in a magnetic composition or material.

22. A process according to claim 8, comprising, after the stages (A), (B), (C) and (D),
a stage (G1) which consists in reacting said dispersion as claimed in one of claims 1 to 7 with chemical entities E capable of forming a bond with the aminated groups R present at the surface of the particles (p), at a pH of less than 8, thus leading to a dispersion according to claim 1, modified in surface by said entities E

23. The process of claim 22, wherein the chemical entities E exhibit aldehyde groups, in that the aminated groups R exhibit -NH₂ groups and in that stage (G1) consists in reacting the dispersion as claimed in one of claims 1 to 7 with chemical entities E carrying -CHO groups in the presence of a reducing agent.

24. The process of claim 23, wherein the entities E are molecules of polysaccharides, a portion of the -OH groups of which have been oxidized to give -CHO groups.

25. The process of claim 24, wherein the entities E are dextran molecules, a portion of the -OH groups of which have been oxidized to give -CHO groups.

26. An aqueous dispersion according to any one of claims 1 to 7, wherein chemical entities E, which are polysaccharides, for example dextran molecules wherein a portion of the -CH groups have been oxidized to give -CHO groups, are immobilized on the modified surface of the particles, said dispersion as obtained as claimed in the process of any one of claims 22 to 25.

27. The dispersion of claim 26, wherein at least 90% by number of the solid components which it comprises are separate particles comprising a single central core based on a magnetic iron oxide having dimensions of less than 20 nm.

28. The aqueous dispersion of particles according to claim 27, which is an aqueous dispersion based on a magnetic iron oxide at the surface of which are immobilized, by covalent bonding, molecules of polysaccharides bonded to the surface via covalent bonds of formula -NH-CH₂-, as obtained by the process of claim 24.

29. The aqueous dispersion according to claim 27, which is an aqueous dispersion of particles based on a magnetic iron oxide at the surface of which are immobilized, by covalent bonding, dextran molecules via covalent bonds -NH-CH₂-, as obtained by the process of claim 25.

30. The dispersion according to any one of claim 28 or claim 29, wherein the average hydrodynamic diameter of the particles with a surface modified by the molecules of polysaccharides is less than 50 nm.

31. The dispersion according to any one of claims 28 to 30, wherein at least 90% of the solid components in suspension are separate particles comprising a single central core, based on an iron oxide, having dimensions of less them 20 nm, this core being surrounded by a layer comprising the covalently bonded molecules of polysaccharides.

32. The dispersion of claim 28 to 31, wherein the particles based on magnetic iron oxide are essentially composed of maghemite (γ-Fe₂O₃).

33. The dispersion according to any one of claims 28 to 32, wherein a portion of the -OH groups of the molecules of polysaccharides immobilized at the surface of the particles are oxidized in the form of -CHO groups.

34. Use of the dispersion according to any one of claims 28 to 33, for the preparation of composition of contrast agents for magnetic resonance imaging.

35. A composition of contrast agents for magnetic resonance imaging, comprising the dispersion of any one of claims 28 to 33.

36. Process according to claim 24, further comprising a stage (G2) which consists in reacting said dispersion of claim 33 with chemical entities F capable of forming a bond with the molecules of polysaccharides.

37. The process of claim 36, wherein the stage (G2) consists in reacting a dispersion as claimed in claim 33 with chemical entities F having an -NH₂ group and in treating the medium obtained with a reducing agent.

38. An aqueous dispersion of particles according to claim 26, wherein said aqueous dispersion of particles is based on a magnetic iron oxide at the surface of which are immobilized, by covalent bonding, dextran molecules via covalent bonds of formula -NH-CH₂-, these dextran molecules being themselves bonded to chemical entities F, this dispersion being capable of being obtained as claimed in the process of claim 36 or claim 37.

39. A composition of a contrast agent for medical imaging having an affinity for given cells, tissues or organs, **characterized in that** it comprises a dispersion as claimed in claim 38 in which the entities F are entities exhibiting an affinity with regard to said cells, said tissues or said organs.

40. A composition for therapeutic use, which comprises a dispersion as claimed in claim 38 in which the entities F are therapeutic active principles.

41. The composition of claim 39 or claim 40, in the form of an injectables composition.

42. Use of a dispersion according to any one of claims 1 to 7,for the preparation of a suspension of essentially separate magnetic particles at the surface of which chemical entities are immobilized, where the immobilization of the chemical entities is achieved by establishing a bond between said chemical entities and the aminated groups R present at the surface of the particles (p).

## Patentansprüche

1. Wässrige Dispersion umfassend Partikel (p) auf der Basis eines magnetischen. Eisenoxids mit Abmessungen kleiner oder gleich 20 nm, deren Oberfläche durch Pfropfen von Aminogruppierungen R modifiziert ist, die in kovalenter Weise an die Oberfläche der Partikel gebenden sind, bei der der isoelektrische Punkt der so modifizierten Oberfläche großer oder gleich 10 ist.

2. Wässrige Dispersion nach Anspruch 1, **dadurch gekennzeichnet, dass** sie einen pH-Wert kleiner oder gleich 8 aufweist, und dass sie sich in der Form einer Dispersion, von Partikeln präsentiert, die einen mittlere hydrodynamischen Durchmesser von kleiner oder gleich 20 nm aufweisen.

3. Wässrige Dispersion nach Anspruch 2, **dadurch gekennzeichnet, dass** der mittlere hydrodynamische der Partikel (p) zwischen 3 und 15 nm liegt.

4. Wässrige Dispersion nach einem beliebigen der Ansprüche 1 bis 3, **dadurch gekennzeichnet, dass** die Partikel (p) im Wesentlichen aus, vorzugeweise monokristallinem, Maghemit (Fe₂O₃-γ) bestehen.

5. Wässrige Dispersion, nach einem beliebigen der Ansprüche 1 bis 4, **dadurch gekennzeichnet, dass** die Aminogruppierungen R Gruppierungen der Formel -(A)-NH₂ sind, in der die Gruppierung -(A)- eine Kohlenwasserstoffkette bezeichnet, die 1 bis 12 Kohlenstoffatome aufweist, gegebenenfalls unterbrochen durch eine oder mehrere -NH-Gruppierungen.

6. Wässrige Dispersion nach Anspruch 5, **dadurch gekennzeichnet**, das die Aminogruppierungen ausgewählt sind aus:
(i) den Gruppierungen der Formel -(CH₂)n₁-NH₂, wobei n₁-1, 2, 3, 4, 5, 6, 7 oder 8 ist;
(ii) den Gruppierungen der Formel -(CH₂)n₂-NH-(CH₂)n₂,-NH₂, wobei n₂ und n₂- unterschiedlich oder identisch sind und jeweils 1, 2, 3, 4, 5 oder 6 bezeichnen, wobei klar ist, dass (n₂+n₂) zwischen 2 und 9 verbleibt;
(iii) den Gruppierungen der Formel -(CH₂)n₃-NH-(CH₂)n_{3'}-NH-(CH₂)n_{3"}-NH₂, wobei n₃, n_{3'} und n_{3"} unterschiedlich oder identisch sind und jeweils 1, 2, 3 oder 4 bezeichnen, wobei klar ist, dass (n₃+n_{3'}+n_{3"},) zwischen 3 und 12 verbleiben.

7. Wässrige Dispersion nach einem beliebigen der Ansprüche 1 bis 6, **dadurch gekennzeichnet, dass** die Aminogruppierungen R an die Fläche der Partikel (p) über eine Verbindung: 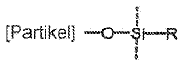 gebunden sind.

8. Verfahren zur Herstellung einer Dispersion nach einem beliebigen der Ansprüche 1 bis 7, die Schritte umfassend, die darin bestehen:
(A) Bereitstellen einer wässerigen Sauredispersion von Partikeln (p₀) auf der Basis eines magnetischen Eisenoxids von Abmessungen kleiner als 20 nm, wobei die Dispersion in saurem Medium eine kolloidale Stabilität mindestens in einem pH-Bereich aufweist, wobei die Stabilität derart ist, dass in deren pH-Bereich eine Dispersion an Partikeln beobachtet wird, die einen mittleren hydrodynamischen Durchmesser kleiner als 20 nm haben, ohne ein Rühren aufrechterhalten zu müssen, wobei weniger als 5% der Anzahl von festen, in Suspension beobachteter Stoffe Agglomerate mehrerer Partikel sind;
(B) Kontaktieren der kolloidalen Säuredispersion des Schritts (A) mit Silanen der Formel R-SiX₁X₂X₃, wobei
- R eine Aminogruppierung bezeichnet, wie sie in Anspruch 1 oder einem der Ansprüche 5 oder 6 definiert sind,
- X₁, X₂ und X₃ identische oder unterschiedliche Gruppierungen sind, die jede eine hydrolysierbare Gruppe in einem sauren Medium bezeichnet,
wobei das in Kontakt Bringen durchgeführt wird, indem das Medium in dem pH-Bereich gehalten wird, in dem die kolloidale Stabilität der Dispersion sichergestellt wird;
(C) Hinzufügen eines wasserlöslichen Benetzungsmittels von einer Siedetemperatur größer als Wasser zu dem reaktiven Medium, dann Aufheizen des reaktiven Mediums auf eine Temperatur, die ausreicht, um das Wasser, aber nicht das Benetzungsmittel zu eliminieren; und
(D) Zurückgewinnen der am Ende des Schritts (C) erhaltenen Partikel und Dispergierten derselben in einem wässrigen Medium.

9. Verfahren nach Anspruch 8, **dadurch gekennzeichnet, dass** die wässrig, kolloidale Säuredispersion des Schritts (A) derart ist, dass in dem pH-Bereich, in dem die kolloidale Stabilität sichergestellt ist, der mittlere hydrodynamische Durchmesser der Partikel, die in Suspension beobachtet werden, zwischen 3 und 15 nm liegt.

10. Verfahren nach Anspruch 8 oder nach Anspruch 9, **dadurch gekennzeichnet, dass** die wässrige, kolloidale Säuredispersion des Schritts (A) derart das in dem pH-Bereich, in dem die kolloidale Stabilität sichergestellt ist, weniger als 5% der Anzahl der festen Stoffe, die in Suspension beobachtet werden, Agglemerate von mehreren Partikeln sind.

11. Verfahren nach einem beliebigen der Ansprüche 8 bis 10, **dadurch gekennzeichnet, dass** die im Schritt (B) verarbeiteten Silane Amino-Trialkoxysilane der Formel R-Si(OR') (OR") (OR"') sind, in der:
- R eine Aminogruppe ist, wie sie in Anspruch 8 definiert wurde; und
- R', R" und R"' unterschiedlich oder identisch sind und jeweils eine Alkylgruppierung bezeichnen, die 1 bis 5 Kohlenstoffatome umfassen.

12. Verfahren nach Anspruch 11, **dadurch gekennzeichnet, dass** die Silane des Schrittes (B) ausgewählt werden unter:
- dem γ-Aminopropyltrimethoxysilan der Formel;
(CH₃O)₃-Si-(CH₂)₃-NH₂;
- dem N-β-Aminoethyl-γ-aminopropyltrimethoxysilan der Formel:
(CH₃O₃)₃-Si-(CH₂)₃-NH-(CH₂)₂-NH₂;
- dem N'-β-Aminoethyl-N-β-aminoethyl-γ-aminopropyltrimethoxysilane der Formel:
(CH₃O)₃-Si-(CH₂)₃-NH-(CH₂)₂-NH-(CH₂)₂-NH₂.

13. Verfahren nach einem beliebigen der Ansprüche 8 bis 12, **dadurch gekennzeichnet, dass** die Silane des Schrittes (B) in Lösung in ein organisches Lösungsmittel eingeführt, werden, dass das Benetzungsmittel des Schritts (C) in dem organischen Lösemittel lösbar ist, das die in Schritt (B) eingeführten Silane gelöst hat und eine Siedetemperatur besitzt, die höher als die des organischen Lösungsmittels ist, und dass der Schritt (c) einen Schritt der Aufheizung auf eine Temperatur umfasst, die ausreicht, um das organische Lösungsmittel zu eliminieren ohne das Benetzungsmittel zu eliminieren.

14. Verfahren nach einem beliebigen der Ansprüche 8 bis 13, **dadurch gekennzeichnet, dass** in dem Schritt (C) das Benetzungsmittel Glyzerin ist.

15. Verfahren nach einem beliebigen der Ansprüche 8 bis 14, **dadurch gekennzeichnet, dass** die Aufheizung im Schritt (C) unter Vakuum durchgeführt wird.

16. Verfahren nach Anspruch 15, **dadurch gekennzeichnet, dass** die Dehydratisierung des Schritts (c) bei einer Temperatur kleiner oder gleich 130°C durchgeführt wird.

17. Verfahren nach einem beliebigen der Ansprüche 8 bis 16, **dadurch gekennzeichnet, dass** der Schritt (D) eine Spülung der am Ausgang des Schritts (C) erhaltenen Partikel umfasst, die durchgeführt wird, ohne die Partikel trocknen zu lassen, gefolgt von einer Dispersion des nicht getrockneten Niederschlags an Partikeln, der in wässrigem Medium verhalten wurde.

18. Verfahren nach einem beliebigen der Ansprüche 8 bis 17, **dadurch gekennzeichnet, dass** die Dispersion der Partikel, die bei dem Schritt (D) bewirkt wird, derart realisiert wird, dass die am Ausgang des Schritts (C) wiedergewonnenen Partikel in Wasser gegeben werden und progressiv der pH-Wert des Mediums durch langsames Zufügen einer Säure verringert wird.

19. Verwendung einer Dispersion nach einem beliebigen der Ansprüche 1 bis 7 für die Herstellung einer Zusammensetzung, die auf oralem oder parenteralem Wege beim Menschen und beim Tier anwendbar ist, und insbesondere für die Herstellung von injizierbaren Zusammensetzungen von Kontrastmittel für die Bildherstellung durch magnetische Resonanz.

20. zusammensetzung für eine Applikation beim Menschen oder beim Tier, umfassend eine Dispersion nach einem beliebigen der Ansprüche 1 bis 7.

21. Verwendung einer Dispersion nach einem beliebigen der Ansprüche :1 bis 7 zum Zwecke der magnetischem Ladung in einer Zusammensetzung oder einem magnetischen Material.

22. Verfahren nach Anspruch 8, umfassend folgend auf die Schritte (A), (B), (C) und (D) einen Schritt (G1), der darin besteht, die Dispersion nach einen der Ansprüche 1 bis 7 mit chemischen Stoffen E reagieren zu lassen, die dazu neigen, eine Verbindung mit den Aminogruppierungen R zu bilden, die an der Fläche der Partikel (p) vorhanden sind, bei einem pH-Wert kleiner als 8. wodurch eine Dispersion nach Anspruch 1 erhalten wird, die an der Oberfläche durch die Stoffe E modifiziert ist.

23. Verfahren nach Anspruch 22, **dadurch gekennzeichnet, dass** die chemischen Stoffe E Aldehydgruppierungen auf weisen, dass die Aminogruppierungen R Gruppierungen -NH₂ aufweisen und dass der Schritt (Gl) darin besteht, die Dispersion nach einem der Ansprüche 1 bis 9 mit den chemischen Stoffen E in Anwesenheit eines Reduktionsmittels reagieren zu lassen, die Träger von Gruppierungen -CHO sind.

24. Verfahren nach Anspruch 23, **dadurch gekennzeichnet, dass** die Stoffe E Moleküle von Polysaccharinden sind, von denen ein Teil der Gruppierungen -OH in einer Gruppierung -CHO oxidiert wurde.

25. Verfahren nach Anspruch 24, **dadurch gekennzeichnet, dass** die Stoffe E Dextranmoleküle sind, von denen ein Teil der Gruppierungen -OH in Gruppierungen -CHO oxidiert wurde,

26. Wässrige Dispersion nach einem beliebigen der Ansprüche 1 bis 7, in der die chemische Stoffe E, die Polysaccharide sind, beispielsweise von Dextranmolekülen, von denen ein Teil der Gruppierungen -OH in Gruppierungen -CHO oxidiert wurde, an der modifizierten Oberfläche der Partikel immobilisiert werden, wobei die Dispersion geeignet ist, nach dem Verfahren nach einem beliebigen der Ansprüche 22 bis 25 erhalten zu werden.

27. Dispersion nach Anspruch 26, **dadurch gekennzeichnet, dass** mindestens 90% der Anzahl der festen Elemente, die sie enthält, individualsierte Partikel sind, die einen einzigen zentralen Kern auf der Basis von magnetischem Eisenoxid umfassen, der Abmessung kleiner 20 nm aufweist.

28. Wässerige Dispersion nach Anspruch 27, die eine wässrige Dispersion von Partikeln auf der Basis eines magnetischer Eisenoxids ist, auf deren Oberfläche durch kovalent Bindung Polysaccharidmoleküle immobilisiert sind, die an die Fläche über kovalente Bindungen der Formel -NH-CH₂- gebunden sind, wobei die Suspension geeignet ist, nach dem Verfahren nach Anspruch 24 erhalten zu werden.

29. Wässrige Dispersion nach Anspruch 27, die eine wässrige Dispersion von Partikeln auf der Basis eines magnetischen Eisenoxids ist, auf deren Oberfläche durch kovalente Bindung Dextranmoleküle über kovalenten Bindungen -NH-CH₂- immobilisiert: sind, wobei diese Dispersion geeignet ist, nach dem Herfahren nach Anspruch 25 erhalten zu werden.

30. Wässrige Dispersion nach Anspruch 28 oder nach Anspruch 29, bei der der mittlere hydrodynamische Durchmesser der Partikel der von den Polysaccharidmolekülen modifizierten Oberfläche kleiner als 50 nm ist.

31. Wässrige Dispersion nach einem beliebigen der Ansprüche 28 bis 30, bei der mindestens 90% der festen in suspension vorhandenen Elemente individualisierte Partikel sind, die einen einzigen zeritralen Kern auf der Basis eines Eisenoxids umfassen, der Abmessungen kleiner als 20 nm aufweist, wobei dieser Kern von einer Schicht umgeben ist, die in kovalenter Weise gebundene Polysaccharidmoleküle umfasst.

32. Dispersion nach einem beliebigen der Ansprüche 28 bis 31, bei der die Partikel auf der Basis von magnetischem Eisenoxid im Wesentlichen aus Maghemit (Fe₂O₃-γ) gebildet sind.

33. Dispersion nach einem beliebigen der Ansprüche 28 bis 32, bei dem ein Teil der Gruppierungen -OH der Polysaccharidmoleküle, die an der Partikelfläche immobilisiert sind, in Form von Gruppierungen -CHO oxidiert sind.

34. Verwendung einer Dispersion nach einem beliebigen der Anspräche 28 bis 33 für die Herstellung einer Kontrastmittelzusammensetzung für die Bildherstellung durch magnetische Resonanz.

35. Zusammensetzung von Kontrastmitteln für die Bildherstellung durch magnetische Resonanz, umfassend eine Dispersion nach einem beliebigen der Ansprüche 28 bis 33.

36. Verfahren nach Anspruch 24, außerdem einen Schritt (C2) umfassend, der darin besteht, eine Dispersion nach einem beliebigen der Ansprüche 28 bis 33 mit chemischen Stoffen F reagieren zu lassen, die geeignet sind, eine Bindung mit den Polysaccharidmolekülen einzugehen,

37. Verfahren nach Anspruch 36, bei dem der Schritt (G2) darin besteht, eine Dispersion nach Anspruch 33 mit chemischen Stoffen F reagieren zu lassen, die eine Gruppierung -NH₂ aufweisen, und das erhaltene Medium mit einem Reduktionsmrittel zu behandelt.

38. Wässrige Dispersion von Partikeln nach Anspruch 26, **dadurch gekennzeichnet, dass** sie auf Basis eines magnetischen Eisenoxids ist, auf deren Oberfläche durch kovalente Bindung Dextranmoleküle über kovalente Bindungen der Formel -NH-CH2- immobilisiert sind, wobei diese Dextranmoleküle selbst, an chemische Stoffe F gebunden sind, wobei diese Dispersion geeignet ist, nach dem Verfahren der Ansprüche 36 oder 37 erhalten zu werde.

39. Zusammensetzung eines Kontrastmittels für die medizinische Bildherstellung, die eine Affinität für Zellen, Gewebe oder vorgegebene Organe aufweist, **dadurch gekennzeichnet, dass** sie eine Dispersion nach Anspruch 38 umfasst, in der die Stoffe F Stoffe sind, die eine Affinität gegenüber den Zellen, den Gewebten oder den Organen aufweisen.

40. Zusammensetzung zur therapeutischen Anwendung, **dadurch gekennzeichnet, dass** sie eine Dispersion nach Anspruch 38 umfasst, in der die Stoffe F therapeutisch aktive Stoffe sind.

41. Zusammensetzung nach Anspruch 39 oder nach Anspruch 40, **dadurch gekennzeichnet, dass** sie die Form einer injizierbaren Zusammensetzung aufweist.

42. Verwendung einer Dispersion nach einem beliebiges der Ansprüche 1 bis 7 für die Herstellung einer Suspension aus magnetischen, hauptsächlich individualisierten Partikeln, an deren Oberfläche chemische Stoffe immobilisiert sind, wobei die Immobilisierung der chemischen. Stoffe dadurch realisiert wird, dass eine Bindung zweischen den chemischen Stoffen und den Aminogruppierungen R hergestellt wird, die auf der Oberfläche der Partikel (p) vorhanden sind.
